(19) Europäisches Patentamt
European Patent Office
Office européen des brevets

(11) **EP 3 261 632 B1**

(12) **EUROPEAN PATENT SPECIFICATION**

(45) Date of publication and mention
of the grant of the patent:
**31.07.2019  Bulletin 2019/31**

(51) Int Cl.:
**A61K 31/197** (2006.01)    **A61P 25/00** (2006.01)
**A61K 45/06** (2006.01)

(21) Application number: **16756494.7**

(22) Date of filing: **26.02.2016**

(86) International application number:
**PCT/US2016/019900**

(87) International publication number:
**WO 2016/138454 (01.09.2016 Gazette 2016/35)**

(54) **DIETARY SUPPLEMENTATION FOR IMPROVING PHYSIOLOGICAL RESPONSES RELATED TO POST-TRAUMATIC STRESS DISORDER**

NAHRUNGSERGÄNZUNG ZUR VERBESSERUNG DER PHYSIOLOGISCHEN REAKTIONEN IM ZUSAMMENHANG MIT POSTTRAUMATISCHER BELASTUNGSSTÖRUNG

COMPLÉMENTATION ALIMENTAIRE POUR L'AMÉLIORATION DES RÉPONSES PHYSIOLOGIQUES ASSOCIÉES À UN TROUBLE DE STRESS POST-TRAUMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2015  US 201562126094 P**

(43) Date of publication of application:
**03.01.2018  Bulletin 2018/01**

(73) Proprietor: **Natural Alternatives International, Inc.
Carsbad, California 92008 (US)**

(72) Inventor: **HOFFMAN, Jay R.
Oviedo, FL 32766 (US)**

(74) Representative: **Hamer, Christopher K. et al
Mathys & Squire LLP
The Shard
32 London Bridge Street
London SE1 9SG (GB)**

(56) References cited:
**WO-A2-2009/033755      US-A1- 2014 079 823
US-A1- 2015 057 354**

- **HOFFMAN JAY R ET AL: "[beta]-Alanine supplemented diets enhance behavioral resilience to stress exposure in an animal model of PTSD", AMINO ACIDS, SPRINGER VERLAG, AU, vol. 47, no. 6, 11 March 2015 (2015-03-11) , pages 1247-1257, XP035889281, ISSN: 0939-4451, DOI: 10.1007/S00726-015-1952-Y [retrieved on 2015-03-11]**
- **TATSURO MURAKAMI ET AL: "The impact of taurine- and beta-alanine-supplemented diets on behavioral and neurochemical parameters in mice: antidepressant versus anxiolytic-like effects", AMINO ACIDS ; THE FORUM FOR AMINO ACID AND PROTEIN RESEARCH, SPRINGER-VERLAG, VI, vol. 39, no. 2, 23 January 2010 (2010-01-23), pages 427-434, XP019853778, ISSN: 1438-2199**
- **NITSAN KOZLOVSKY ET AL: "Long-term down-regulation of BDNF mRNA in rat hippocampal CA1 subregion correlates with PTSD-like behavioural stress response", INTERNATIONAL JOURNAL OF NEUROPSYCHOPHARMACOLOGY, vol. 10, no. 06, 12 February 2007 (2007-02-12), XP055505639, Cambridge ISSN: 1461-1457, DOI: 10.1017/S1461145707007560**

**(Cont. next page)**

- SHIRO TAKEI ET AL: "Enhanced hippocampal BDNF/TrkB signaling in response to fear conditioning in an animal model of posttraumatic stress disorder", JOURNAL OF PSYCHIATRIC RESEARCH, ELSEVIER LTD, GB, vol. 45, no. 4, 23 August 2010 (2010-08-23), pages 460-468, XP028367246, ISSN: 0022-3956, DOI: 10.1016/J.JPSYCHIRES.2010.08.009 [retrieved on 2010-09-01]

- MURAKAMI ET AL.: 'The impact of taurine- and beta-alanine-supplemented diets on behavioral and neurochemical parameters in mice: antidepressant versus anxiolytic-like effects' AMINO ACIDS vol. 39, no. 2, 23 January 2010, pages 427 - 434, XP019853778

## Description

### FIELD OF THE INVENTION

The present invention relates to dietary supplementation and physiology, and, more specifically, to methods of improving physiological responses related to post-traumatic stress disorder (PTSD).

### BACKGROUND OF THE INVENTION

Investigations examining β-alanine (herein also referred to as beta-alanine or BA) ingestion have been consistent in demonstrating significantly enhanced athletic performance during high intense activity (*e.g.*, resistance exercise, repeated sprints) to a greater magnitude than a placebo (Hill *et al.,* 2007; Hoffman *et al.,* 2006; 2008a; 2008b; 2012; Kendrick *et al.,* 2008; Stout *et al.,* 2006; 2007). The efficacy of β-alanine ingestion appears centered on its ability to enhance the quality of a workout and sport performance by delaying skeletal muscle fatigue when supplemented in an effective amount over a sufficient period of time as with a dietary supplement. The ergogenic properties of β-alanine taken as a single dose by itself appear to be very limited, but when consumed in sufficient dosages over time, β-alanine combines in the skeletal muscle with L-histidine to form the dipeptide carnosine (beta-alanylhistidine) and appear to have ergogenic effects (Dunnett and Harris, 1999). The primary role of carnosine is in the maintenance of acid-base homeostasis through enhanced intra-muscular hydrogen ion ($H^+$) buffering capacity (Harris *et al.,* 2006). Increasing intra-muscular carnosine concentration through β-alaninesupplementation has demonstrated ergogenic potential for maximal exercise lasting 60 sec - 240 sec (Hobson *et al.,* 2012). Because carnosine is located in other tissues in addition to skeletal muscle, such as the brain and heart, it may also have additional physiological roles.

Previous research has shown β-alanineto have beneficial effects on brain function related to depression and anxiety-like behavior.

Several studies have suggested that carnosine may serve as a neuroprotector (Boldyrev *et al.,* 2010; Stout *et al.,* 2008). Carnosine's biological role as an antioxidant, an antiglycating and ion-chelating agent suggests that it may have a potential role in neuroprotection during oxidative stress. In addition, recent research has demonstrated that β-alanine can increase carnosine concentrations in the brain resulting in a decrease in serotonin concentrations, increase brain-derived neurotrophic factor (involved in the growth and differentiation of new neurons and synapses), and provide possible antianxiety-like effects (Murakami and Furuse, 2010). Thus, increases in carnosine concentration in the brain may also provide a benefit in maintaining focus, alertness and cognitive function during and after highly fatiguing, high intense activity.

Stress resulting from an acute traumatic experience can result in a variety of manifestations that include recurring and unwanted recollections or dreams of the event that cause significant behavioral changes (American Psychiatric Association, 2013). Responses from acute stress may include avoidance of feelings or reminders of the event, marked arousal including irritability, hypervigilance, an elevated startle response, a concentration deficit or emotional numbing (American Psychiatric Association, 2013). Although these responses to acute stress are often used to diagnose post-traumatic stress disorder (PTSD) (American Psychiatric Association, 1994), several studies have indicated that some individuals are able to adapt within a short time period following the traumatic experience and not experience PTSD (Bisson et al., 2004; Bryant et al., 2008). Identification of an acute stress disorder within the first month of the traumatic experience appears to be only moderately sensitive (51% and 45% within 3 and 12 months, respectively) for predicting PTSD (Bryant et al., 2014). The absence of acute stress disorder, however, appears to be highly predictive (95% and 94% within 3 and 12 months, respectively) that the individual will not develop PTSD (Bryant et al., 2014).

The pathophysiology of PTSD is thought to be related to changes in the structure of neurons within areas of the brain that control stress and memory (McEwen, 2007). The hippocampus is one of the most sensitive and adaptable regions of the brain, and many of the changes within the hippocampus occur within the dentate gyrus (DG) - CA3 region (McEwen, 2007). The DG - CA3 region is thought to play a role in the memory of sequences of events, and several changes have been suggested to occur in this region during stress such as a suppression of neurogenesis or cell survival, and a degeneration of dendrites and synapses (McEwen, 1999; Sousa et al., 1998; Stewart et al., 2005). The effect of stress on brain function though is not limited to the hippocampus only. The hippocampus works synergistically with the cortex and amygdala to control the processing of emotional memories (Richardson et al., 2004) and reduction of fear (Milad and Quirk, 2002). Although the mechanism that is stimulating the structural remodeling of neurons in the brain is quite complex, evidence is compelling that changes in circulating glucocorticoids (McEwen, 2007; Myers et al., 2014) and expression of brain-derived neurotrophic factor (BDNF) (Yao et al., 2011) are closely linked to the plasticity of brain function.

Stress has a profound influence on changes in the neuroendocrine system resulting in a significant elevation in glucocorticoids (Myers et al., 2014). Glucocorticoids are released from the adrenal gland and cross the blood-brain barrier to bind to receptors in neurons or glia cells. Glucocorticoid receptors are found in high concentrations within the brain, specifically in areas that are related to sites of stress such as the hippocampus, amygdala and frontal cortex (Fuxe et al., 1987; Myers et al., 2014). Corticosterone injections in the dorsal hippocampus of rodents have been demonstrated to cause PTSD-like memory impairments that accompany impaired hippocampal function (Kaouane et al., 2012). Elevations in glucocorticoids are generally associated with dendritic remodeling and memory that often resemble what is seen during chronic stress (Miller and McEwen, 2006). The glucocorticoid response to stress appears, however, to follow an inverted U pattern. Very low or very high concentrations of circulating glucocorticoids during periods of stress are more likely to negatively alter neural plasticity (Miller and McEwen, 2006).

BDNF is part of the neurotrophin family, and has been demonstrated to have an important role in neuronal remodeling and modulating synaptic plasticity and neurotransmitter release (Castrén and Rantamäki, 2010). BDNF concentrations in individuals diagnosed with PTSD have been compared to individuals who were exposed to a traumatic event but not diagnosed with PTSD (Angelucci et al., 2014). Serum BDNF concentrations were significantly lower in PTSD patients compared to the control subjects. In a rodent model, exposure to stress has been shown to down-regulate BDNF mRNA expression (Kozlovksy et al., 2007). Increasing BDNF expression, or decreasing glucocorticoid levels in individuals who are experiencing stress, may provide increased resiliency to PTSD following exposure to trauma.

Treatment for PTSD is quite varied and generally involves a combination of psychotherapy and pharmacological options that occur following diagnosis (Kirkpatrick and Heller, 2014). Less information is available, however, regarding potential options for preventing or increasing resiliency to PTSD. A β-alaninesupplemented diet in mice was able to increase brain carnosine concentrations in the cerebral cortex and hypothalamus, and increase the concentration of BDNF in the hippocampus (Murakami and Furuse 2010). These changes were also accompanied by a significantly greater activity of the mice in the open arms of an elevated plus-maze test. Although β-alanineis considered to act as an inhibitory neurotransmitter that can cross the blood-brain barrier (Takeuchi et al., 2000), the lack of any significant change in β-alanineconcentrations in the cerebral cortex and hypothalamus suggests that the anxiolytic effects observed are likely related to an elevation in brain carnosine (β-alanyl-L-histidine) concentrations. The patent document US 2014/079823 discloses a composition comprising the amino acids L-arginine, oxo-proline, L-lysine, N-acetyl L-cysteine and L-glutamine and Schizonepta powder for use in treating sleep-related symptoms of post-traumatic stress disorder (PTSD) by increasing human growth hormone (hGH) levels.

Reference is made to the following art. Artioli G.G., Gualano B., Smith A., Stout J.R., Junior A.H.L., The Role of β-alanine Supplementation on Muscle Carnosine and Exercise Performance. Med Sci Sports Exerc 42(6):1162-1173, 2010; Baguet, A., et al. (2010). "Important role of muscle carnosine in rowing performance." J of App. Physio. 109(4): 1096-1101; Boldyrev A.A., Stvolinsky S.L., Fedorova T.N., Suslina Z.A., (2010) Carnosine as a natural antioxidant and geroprotector: from molecular mechanisms to clinical trials. Rejuvenation Res. 13:156-158; Cassler N.M., Sams R., Cripe P.A., McGlynn A.F., Perry A.B., Banks B.A., Patterns and perceptions of supplement use by U.S. Marines deployed to Afghanistan. Mil Med. 2013 178(6):659-64; Derave, W., et al. (2010). "Muscle carnosine metabolism and β-alaninesupplementation in relation to exercise and training." Sports medicine 40(3): 247-263; Derave W., Ozdemir M.S., Harris R.C., Pottier A., Reyngoudt H., Koppo K., Wise J.A., Achten E., Beta-Alanine supplementation augments muscle carnosine content and attenuates fatigue during repeated isokinetic contraction bouts in trained sprinters. J Appl Physiol 103(5):1736-1743, 2007; Dunnett M., Harris R.C. (1999), Influence of oral beta-alanine and L-histidine supplementation on the carnosine content of the gluteus medius. Equine Vet J Suppl 30:499-504; Del Favoro S., Roschel H., Solis M., Hayashi A., Artiolo G., Otaduy M., Benatti F., Harris R., Wise J., Leite C., Pereira R., de Sa-Pinto A., Lancha-Junior A., Gualano B. (2012), Beta-alanine (Carnosyn™) supplementation in elderly subjects (60-80 years): effects on muscle carnosine content and physical capacity. Amino Acids 43:49-56; Estrada A., Kelley A.M., Webb C.M., Athy J.R., Crowley J.S., Modafinil as a replacement for dextroamphetamine for sustaining alertness in military helicopter pilots. Aviat Space Environ Med. 2012, 83(6):556-64; Evans R.K., Scoville C.R., Ito M.A., Mello R.P., Upper body fatiguing exercise and shooting performance. Mil. Med. 168:451-456, 2003; Gillingham R.L., Keefe A.A., Tikuisis P., Acute caffeine intake before and after fatiguing exercises improves target shooting engagement time. Aviat. Space Environ. Med. 75:865-871, 2004; Green S.B., Salkind N.J., Akey T.M.,: Using SPSS for Windows: Analyzing and Understanding Data (2nd ed.), Upper Saddle River, NJ: Prentice Hall, 2000; Harman, E. A., et al. (2008). "Effects of two different eight-week training programs on military physical performance." The Journal of Strength & Conditioning Research 22(2): 524-534; Harris R.C., Tallon M.J., Dunnett M., Boobis L., Coakley J., Kim H.J., Fallowfield J.L., Hill C.A., Sale C., Wise J.A., (2006), The absorption of orally supplied β-alanineand its effect on muscle carnosine synthesis in human vastus lateralis. Amino Acids 30(3)279-289; Hayman M., Two minute clinical test for measurement of intellectual impairment in psychiatric disorders. Arch Neuro Psychiatry 1942;47:454-64; Hill C.A., Harris R.C., Kim H.J., Harris B.D., Sale C., Boobis L.H., Kim C.K., Wise J.A., (2007), Influence of β-alaninesupplementation on skeletal muscle carnosine concentrations and high intensity cycling capacity. Amino Acids 32(2):225-233; Hobson R.M., Saunders B., Ball G., Harris R.C., Sale C., (2012), Effects of Beta-alanine supplementation on exercise performance: a meta-analysis. Amino Acids. 43(1):25-37; Hoffman J.R., Emerson N.S., Stout J.R., β-alaninesupplementation. Curr. Sports Med. Rep. 11:189-195, 2012; Hoffman J.R., Ratamess N.A., Faigenbaum A.D., Ross R., Kang J., Stout J.R., Wise J.A., (2008a), Short-duration β-alaninesupplementation increases training volume and reduces subjective feelings of fatigue in college football players. Nutr Res 28(1):31-35; Hoffman J.R., Ratamess N.A., Kang J., Mangine G., Faigenbaum A.D., Stout J.R. (2006), Effect of creatine and β-alanine supplementation on performance and endocrine responses in strength/power athletes. Int J Sport Nutr Exerc Metabab 16(4):430-446; Hoffman J.R., Ratamess N.A., Ross R., Kang J., Magrelli J., Neese K., Faigenbaum A.D., Wise J.A. (2008b), β-Alanine and the hormonal response to exercise. Int J Sports Med 29(12):952-958; Jordan T., Lukaszuk J., Misic M., Umoren J., Effects of beta-alanine supplementation on the onset of blood lactate accumulation (OBLA) during treadmill running: Pre/post 2 treatment experimental design. J Int Soci Sports Nutr 7: 20-27, 2010; Kendrick I.P., Harris R.C., Kim H.J., Kim C.K., Dang V.H., Lam T.Q., Bui T.T., Smith M., Wise J.A. (2008), The effects of 10 weeks of resistance training combined with beta-alanine supplementation on whole body strength, force production, muscular endurance and body composition. Amino Acids. 34(4):547-554; Kern B.D., Robinson T.L., Effects of β-alaninesupplementation on performance and body composition in collegiate wrestlers and football players. J Strength Cond Res. 2011 25:1804-1815; Knapik, J. J., et al. (2004). "Soldier load carriage: historical, physiological, biomechanical, and medical aspects." Military Medicine 169(1): 45-56; Lieberman H.R., Bathalon G.P., Falco C.M., Kramer F.M., Morgan C.A. 3rd, Niro P., Severe decrements in cognition function and mood induced by sleep loss, heat, dehydration, and undernutrition during simulated combat. Biol Psychiatry. 57:422-429, 2005; Lieberman H.R., Stavinoha T.B., McGraw S.M., White A., Hadden L.S., Marriott B.P., Use of dietary supplements among active-duty US Army soldiers. Am J Clin Nutr. 92:985-995, 2010; Lieberman H.R., Tharion W.J., Shukitt-Hale B., Speckman K.L., Tulley R. Effect of caffeine, sleep loss, and stress on cognitive performance and mood during U.S. Navy SEAL training. Psychopharmacology. 164:250-261, 2002; Murakami T., Furuse M. (2010), The impact of taurine- and beta-alanine-supplemented diets on behavioral and neurochemical parameters in mice: antidepressant versus anxiolytic-like effects. Amino Acids. 39:427-434; Nibbeling, N., et al. (2014). "The effects of anxiety and exercise-induced fatigue on shooting accuracy and cognitive performance in infantry soldiers." Ergonomics(ahead-of-print): 1-14; Nindl B.C., Barnes B.R., Alemany J.A., Frykman P.N., Shippee R.L., Friedl K.E., Physiological consequences of U.S. army ranger training. Med. Sci. Sports Exerc. 39:1380-1387, 2007; Nindl, B. C., et al. (2013). "Physiological Employment Standards III: physiological challenges and consequences encountered during international military deployments." European journal of applied physiology 113(11): 2655-2672; Nindl, B. C., et al. (2002). "Physical performance responses during 72 h of military operational stress." Medicine and science in sports and exercise 34(11): 1814-1822; Russo M.B., Arnett M.V., Thomas M.L., Caldwell J.A., Ethical use of cogniceuticals in the militaries of democratic nations. Amer. J. Bioethics. 8:39-49, 2008; Soher, B. J., et al. (1996). "Quantitative proton MR spectroscopic imaging of the human brain." Magnetic resonance in medicine 35(3): 356-363; Stellingwerff, T., et al. (2012). "Effect of two β-alaninedosing protocols on muscle carnosine synthesis and washout." Amino acids 42(6): 2461-2472; Stout J.R., Cramer J.T., Mielke M., O'Kroy J., Torok D.J., Zoeller R.F. (2006), Effects of twenty-eight days of β-alanineand creatine monohydrate supplementation on the physical working capacity at neuromuscular fatigue threshold. J Strength Cond Res 20(4)928-931; Stout J.R., Cramer J.T., Zoeller R.F., Torok D., Costa P., Hoffman J.R., Harris R.C., O'Kroy J. (2007), Effects of β-alanine supplementation on the onset of neuromuscular fatigue and ventilatory threshold in women. Amino Acids. 32(3):381-386; Stout J.R., Graves S.B., Smith A.E., Hartmen M.J., Cramer J.T., Beck T.W., Harris R.C. (2008), The effects of beta-alanine supplementation on neuromuscular fatigue in elderly (55-92 years): a double-blinded randomized study. JISSN. 5(21); Van Thienen R., Van Proeyen K., Vanden Eynde B., Puype J., Lefere T., Hespel P., Beta-alanine, improves sprint performance in endurance cycling. Med Sci Sports Exer 41(4):898-903, 2009; Varley, M.C., Fairweather, I.H., and Aughey, R.J., Validity and reliability of GPS for measuring instantaneous velocity during acceleration, deceleration, and constant motion. J Sports Sci 30: 121-127, 2012; Weeks S.R., McAuliffe C.L., DuRussel D., Pasquina P.F., Physiological and psychological fatigue in extreme conditions: The military example. Phys. Med Rehab. 2:438-441, 2010; Wells A.J., Hoffman J.R., Gonzalez A.M., Stout J.R., Fragala M.S., Mangine G.T., McCormack W.P., Jajtner A.R., Townsend J.R., and Robinson IV, E.H., A Supplement Containing Phosphatidylserine Attenuates Post-Exercise Mood Disturbance and Perception of Fatigue in Humans. Nutrition Research. 33:464-472, 2013; Welsh T.T., Alemany J.A., Montain S.J., Frykman P.N., Young A.J., Nindl B.C., Effects of intensified military field training on jumping performance. Int. J. Sports Med. 29:45-52, 2008. American Psychiatric Association (1994) Diagnostic and Statistical Manual of Mental Disorders. Fourth edition. Washington D.C. American Psychiatric Association. American Psychiatric Association (2013) Diagnostic and Statistical Manual of Mental Disorders. Fifth edition. Washington D.C. American Psychiatric Association. Angelucci F, Ricci V, Gelfo F, Martinotti G, Brunetti M, Sepede G, Signorelli M, Aguglia E, Pettorruso M, Vellante F, Di Giannantonio M, Caltagirone C (2014) BDNF serum levels in subjects developing or not post-traumatic stress disorder after trauma exposure. Brain and Cognition. 84:118-122. Bisson JI, Shepherd JP, Joy D, Probert R, Newcombe RG (2004) Early cognitive-behavioural therapy for post-traumatic stress symptoms after physical injury. Randomized controlled trial. British Journal Psychiatry. 184:63-69. Bonfanti L, Peretto P, De Marchis S, Fasolo A (1999) Carnosine-related dipeptides in the mammalian brain. Progress in Neurobiology. 59:333-353. Bryant RA, Creamer M, O'Donnell M, Silove D, McFarlane AC, Forbes D. (2014) A comparison of the capacity of DSM-IV and DSM-5 acute stress disorder definitions to predict posttraumatic stress disorder and related disorders. Journal of Clinical Psychiatry. Bryant RA, Mastrodomenico J, Felmingham KL, Hopwood S, Kenny L, Kandris E, Cahill C, Creamer M (2008) Treatment of acute stress disorder: a randomized controlled trial. Arch. Gen. Psychiatry. 65:659-703. Castrén E, Rantamäki T (2010) The role of BDNF and its receptors in depression and antidepressant drug action: Reactivation of developmental plasticity. Dev Neurobiol. 70:289-97. Coburn-Litvak PS, Pothakos K, Tata DA, McCloskey DP, Anderson BJ (2003) Chronic administration of corticosterone impairs spatial reference memory before spatial working memory in rats. Neurobiol Learn Mem. 80:11-23. Cohen H, Kozlovsky N, Cramer A, Matar MA, Zohar J (2012a) Animal model for PTSD: From clinical concept to translational research. Neuropharmacology. 62:715-724. Cohen H, Liu T, Kozlovsky N, Kaplan Z, Zohar J, and Mathé AA (2012b) The neuropeptide Y (NPY)-ergic system is associated with behavioral resilience to stress exposure in an animal model of post-traumatic stress disorder. Neuropsychopharmacology. 37:350-363. Cohen H and Zohar J (2004) An animal model of posttraumatic stress disorder: the use of cut-off behavioral criteria. Annals of the New York Academy of Science. 1032: 167-178. Cohen H, Zohar J, Gidron Y, Matar MA, Belkind D, Loewenthal U, Kozlovsky N, Kaplan Z (2006) Blunted HPA axis response to stress influences susceptibility to posttraumatic stress response in rats. Biol. Psychiatry. 59:1208-1218. Cohen H, Zohar J, Matar M (2003) The relevance of differential response to trauma in an animal model of posttraumatic stress disorder. Biol. Psychiatry. 53: 463-473. Cohen H, Zohar J, Matar M A, Zeev K, Loewenthal U, Richter-Levin G (2004) Setting apart the affected: the use of behavioral criteria in animal models of post-traumatic stress disorder. Neuropsychopharmacology. 29: 1962 -1970. Fuxe K, Cintra A, Agnati LF, Harfstrand A, Wikstrom AC, Okret S, Zoli M, Miller LS, Greene JL, Gustafsson JA (1987). Studies on the cellular localization and distribution of glucocorticoid receptor and estrogen receptor immunoreactivity in the central nervous system of the rat and their relationship to the monoaminergic and peptidergic neurons of the brain. Journal of Steroid Biochemistry. 27:159-170. Kaouane N, Porte Y, VAllee M, Brayda-Bruno L, Mons N, Calandreau L, Marighetto A, Piazza PV, Desmedt A (2012) Glucocorticoids can induce PTSD-like memory impairements in mice. Science. 335:1510-1513. Kirkpatrick HA, Heller GM (2014) Post-traumatic stress disorder: Theory and treatment update. International Journal of Psychiatry in Medicine. 47:337-346. Kim H, Yi JH, Choi K, Hong S, Shin KS, Kang SJ (2014) Regional differences in acute corticosterone-induced dendritic remodeling in the rat brain and their behavioral consequences. BMC Neuroscience. 15:65. Kim JJ, Fanselow MS, DeCola JP, Landeira-Fernandez J (1992) Selective impairment of long-term but not short-term conditional fear by the N-methyl-D-aspartate antagonist APV. Behavioral Neuroscience. 106:591-596. Kohen R, Yamamoto Y, Cundy KC, Ames BN (1988) Antioxidant activity of carnosine, homocarnosine, and anserine present in muscle and brain. Proceedings of the National Academy of Sciences. 85:3175-3179. Kozlovsky N, Matar MA, Kaplan Z, Kotler M, Zohar J, Cohen H (2007) Long-term down-regulation of BDNF mRNA in rat hippocampal CA1 subregion correlates with PTSD-like behavioral stress response. International Journal of Neuropsychopharmacology. 10:741-758. Matar MA, Zohar J, Cohen H (2013) Translationally relevant modeling of PTSD in rodents. Cell Tissue Research. 354:127-139. McEwen BS (1999) Stress and hippocampal plasticity. Annual Review of Neuroscience. 22:105-122. McEwen BS (2007) Physiology and neurobiology of stress and adaptation: Central role of the brain. Physiological Reviews. 87:873-904. McFarlane AC, Barton CA, Yehuda R, Wittert G (2011) Cortisol response to acute trauma and risk of posttraumatic stress disorder. Psychoneuroendocrinology. 36:720-727. Milad M, Quirk GJ (2002) Neurons in medial prefrontal cortex signal memory for fear extinction. Nature. 420:70-74. Miller MM, McEwen BS (2006) Establishing an agenda for translational research on PTSD. Annals of the New York Academy of Science. 1071:294-312. Murakami T, Furuse M (2010) The impact of taurine- and beta-alanine-supplemented diets on behavioral and neurochemical parameters in mice: antidepressant versus anxiolytic-like effects. Amino Acids. 39:427-434. Myers B, McKlveen JM, Herman JP (2014) Glucocorticoid actions on synapses, circuits, and behavior: Implications for the energetics of stress. Frontiers in Neuroendocrinology. 35:180-196. Richardson MP, Strange BA, Dolan RJ (2004) Encoding of emotional memories depends on amygdala and hippocampus and their interactions. Nature Neuroscience. 7:278-285. Romeo RD, Waters EM, McEwen BS (2004) Steroid-induced hippocampal synaptic plasticity; sex differences and similarities. Neuron Glia Biology. 1:219-29. Sebastian V, Estil JB, Chen D, Schrott LM, Serrano PA (2013) Acute physiological stress promotes clustering of synaptic markers and alters spine morphology in the hippocampus. Plos One. 8:e79077. Solanki N, Alkadhi I, Atrooz F, Patki G, Salim S (2015) Grape powder prevents cognitive, behavioral, and biochemical impairments in a rat model of posttraumatic stress disorder. Nutrition Research. 35:65-75. Sousa N, Madeira MD, Paula-Barbosa MM (1998) Effects of corticosterone treatment and rehabilitation on the hippocampal formation of neonatal and adult rats. An unbiased stereological study. Brain Research. 794:199-210. Stewart MG, Davies HA, Sandi C, Kraev IV, Rogachevsky VV, Peddie CJ, Rodriguez JJ, Cordero MI, Donohue HS, Gabbott PL, Popov VI (2005) Stress suppresses and learning induces plasticity in CA3 of rat hippocampus: a three dimensional ultrastructural study of thrny excrescences and their postsynaptic densities. Neuroscience. 131:43-54. Takeuchi K, Toyohama H, Sakaquchi M (2000) A hyperosmotic stress-induced mRNA of carp cell encodes NA(+)- and Cl(-)-dependent high affinity taurine transporter. Biochiica et Biophysica Acta. 1464:219-230. Tomonaga S, Kaji Y, Tachibana T, Denbow DM, Furuse M (2005) Oral administration of β-alanine modifies carnosine concentrations in the muscles and brains of chicken. Animal Science Journal. 76:249-254. Tomonaga S, Matsumoto M, Furuse M (2012) β-alanine enhances brain and muscle carnosine levels in broiler chicks. The Journal of Poultry Science. 49:308-312. Tomonaga S, Tachibana T, Takagi T, Saito ES, Zhang R, Denbow DM, Furuse M (2004) Effect of central administration of carnosine and its constituents on behaviors in chicks. Brain Research Bulletin. 63:75-82. Tomonaga S, Yamane H, Onitsuka E, Yamada S, Sato M, Takahata Y, Morimatsu F, Furuse M (2008) Carnosine-induced anti-depressant-like activity in rats. Pharmacology, Biochemistry and Behavior. 89:627-632. Wilson CB, McLaughlin LD, Nair A, Ebenezer PJ, Dange R, Francis J (2013) Inflammation and oxidative stress are elevated in the brain, blood, and adrenal glands during the progression of post-traumatic stress disorder in a predator exposure animal model. Plos One. 8:e76146. Yao SY, Lau BW, Tong JB, Wong R, Ching YP, Qui G, Tang SW, Lee TMC, So KF (2011) Hippocampal neurogenesis and dendritic plasticity support running-improved spatial learning and depression-like behavior in stressed rats. PloS One. 2011; 6:e24263. Zohar J, Yahalom H, Kozlovsky N, Cwikel-Hamzany S, Matar MA, Kaplan Z, Yehuda R, Cohen H (2011) High dose hydrocortisone immediately after trauma may alter the trajectory of PTSD: Interplay between clinical and animal studies. European Neuropsychopharmacology. 21:796-809.

## SUMMARY OF THE INVENTION

The invention relates to a human dietary supplement comprising a free amino acid beta-alanine, or a salt thereof, for use in therapeutically improving physiological responses related to post-traumatic stress disorder. In one embodiment, this is achieved by supplementing the human diet with an effective amount of the free amino acid beta-alanine, or a salt thereof, wherein the effective amount is provided over a period of time. Naturally, a salt of beta-alanine could be taken and would readily convert to the free amino acid in the body or in a suitable delivery medium. In some embodiments of the present invention the free amino acid beta-alanine, or a salt thereof can be administered before, during or after a user experiences symptoms related to PTSD. In other embodiments of the present invention the free amino acid beta-alanine, or a salt thereof can be administered before, during or after a user is diagnosed with PTSD.

The period of time over which the effective amount described herein of beta-alanine is provided may be about 7 days or more. Additionally, the beta-alanine can be given every day over this period of time, may be given on alternative days, or given periodically over this period of time.

Additional features, advantages, and embodiments of the invention are set forth or apparent from consideration of the following detailed description, drawings and claims. It is to be understood that both the foregoing summary of the invention and the following detailed description are exemplary and intended to provide further explanation.

## BRIEF DESCRIPTION OF THE DRAWINGS

The accompanying drawings, which are included to provide a further understanding of the invention and are incorporated in and constitute a part of this specification, illustrate preferred embodiments of the invention and together with the detailed description serve to explain the principles of the invention. In the drawings:

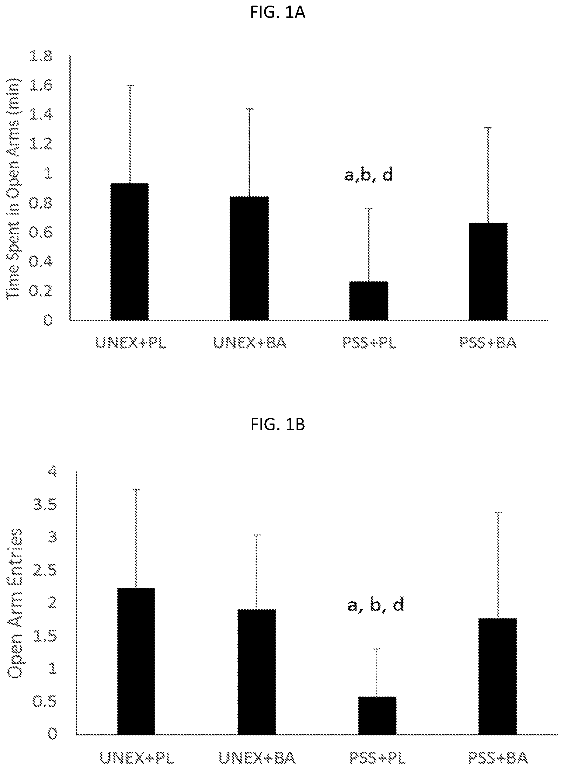

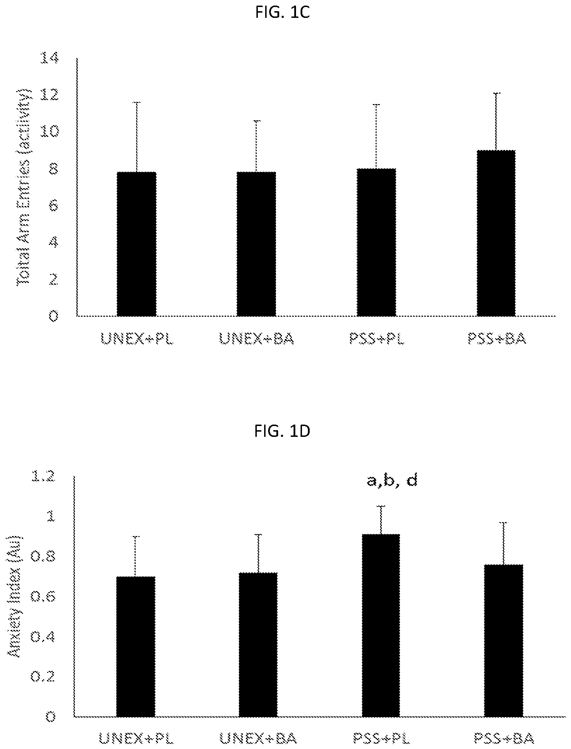

Figs. 1A - 1D are graphs of behavioral performance in elevated plus-maze. Fig. 1A is a comparison between the groups for time spent in open arms; Fig. 1B is a comparison between the groups in the number of open arm entries; Fig. 1C is a comparison between the groups in the total activity; Fig. 1D is a comparison between the groups in the anxiety-index; a = significantly different than UNEX + PL; b = significantly different than UNEX + BA; d = significantly different than PSS + BA; UNEX + PL = animals that were not exposed to the predator scent stress and fed a normal diet; UNEX + BA = animals that were not exposed to the predator scent stress and supplemented with β-alanine; PSS + PL = animals that were exposed to the predator scent stress and fed a normal diet; PSS + BA = animals that were exposed to the predator scent stress and supplemented with β-alanine. All data reported as mean ± SD.

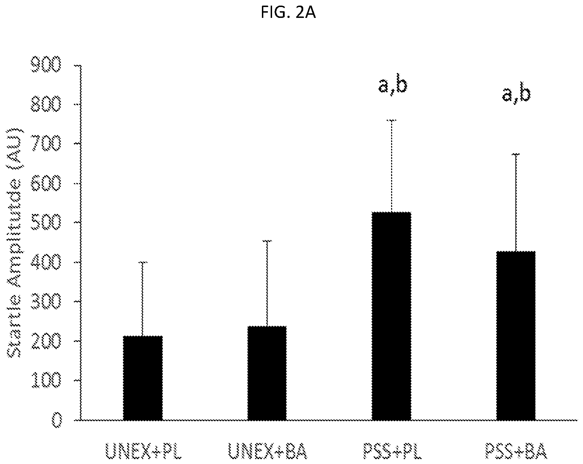

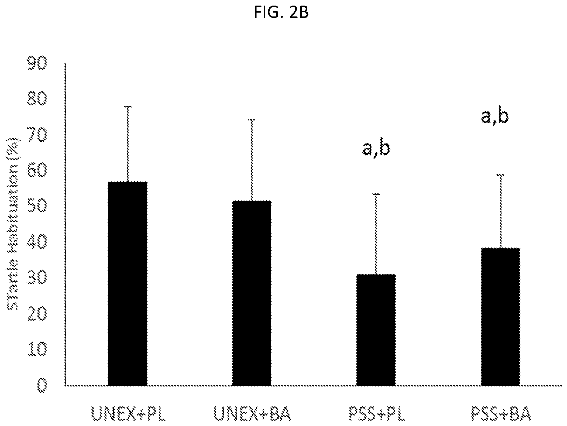

Figs. 2A - 2B are graphs of acoustic startle response. Fig. 2A is a comparison between the groups in startle amplitude; Fig. 2B is a comparison between the groups in startle habituation; a = significantly different than UNEX + PL; b = significantly different than UNEX+ BA; UNEX + PL = animals that were not exposed to the predator scent stress and fed a normal diet; UNEX + BA = animals that were not exposed to the predator scent stress and supplemented with β-alanine; PSS + PL = animals that were exposed to the predator scent stress and fed a normal diet; PSS + BA = animals that were exposed to the predator scent stress and supplemented with β-alanine. All data reported as mean ± SD.

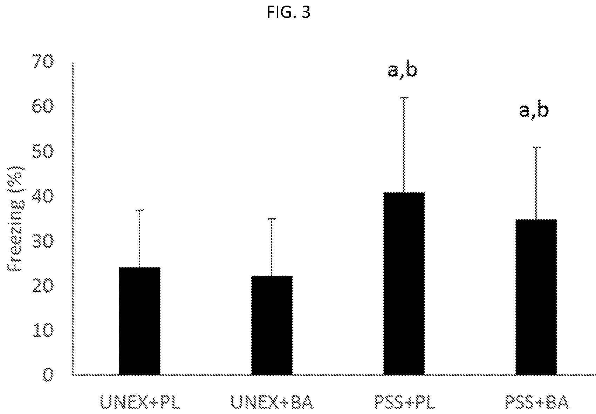

Fig. 3 is a graph of freezing behavior upon cue-exposure; a = significantly different than UNEX + PL; b = significantly different than UNEX+ BA; UNEX + PL = animals that were not exposed to the predator scent stress and fed a normal diet; UNEX + BA = animals that were not exposed to the predator scent stress and supplemented with β-alanine; PSS + PL = animals that were exposed to the predator scent stress and fed a normal diet; PSS + BA = animals that were exposed to the predator scent stress and supplemented with β-alanine. All data reported as mean ± SD.

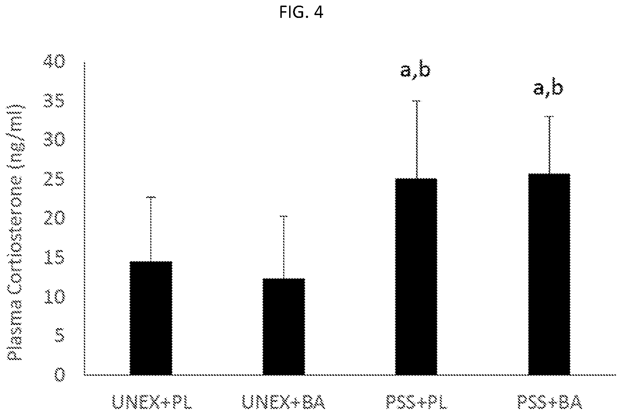

Fig. 4 is a graph of corticosterone concentrations at Day 8 post-PSS exposure; a = significantly different than UNEX + PL; b = significantly different than UNEX+ BA; UNEX + PL = animals that were not exposed to the predator scent stress and fed a normal diet; UNEX + BA = animals that were not exposed to the predator scent stress and supplemented with β-alanine; PSS + PL = animals that were exposed to the predator scent stress and fed a normal diet; PSS + BA = animals that were exposed to the predator scent stress and supplemented with β-alanine. All data reported as mean ± SD.

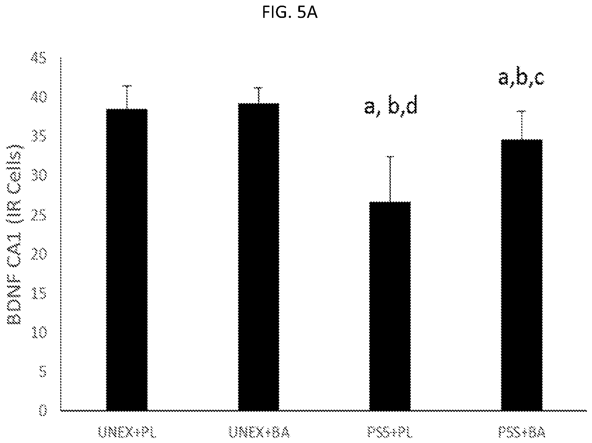

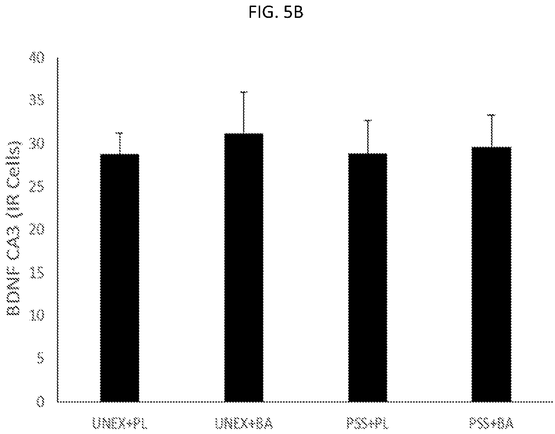

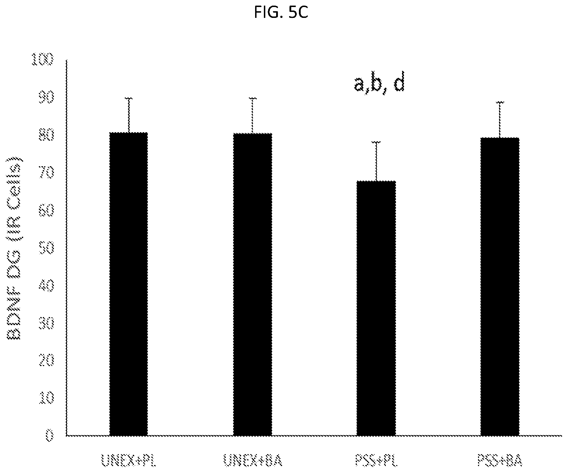

Figs. 5A - 5C are graphs of BDNF expression at Day 8 post-PSS Exposure. Fig. 5A shows BDNF expression in the CA1 subregion, Fig. 5B shows BDNF expression in the CA3 subregion, and Fig. 5C shows BDNF expression in the DG subregion; a = significantly different than UNEX + PL; b = significantly different than UNEX+ BA; c = significantly different than PSS + PL; d = significantly different than PSS + BA; UNEX + PL = animals that were not exposed to the predator scent stress and fed a normal diet; UNEX + BA = animals that were not exposed to the predator scent stress and supplemented with β-alanine; PSS + PL = animals that were exposed to the predator scent stress and fed a normal diet; PSS + BA = animals that were exposed to the predator scent stress and supplemented with β-alanine. All data reported as mean ± SD.

## DETAILED DESCRIPTION OF THE PREFERRED EMBODIMENTS

Methods and embodiments are described for improving physiological responses related to post-traumatic stress disorder (PTSD) through dietary supplementation with the free amino acid beta-alanine, or salt thereof. The methods described herein may be used for many different industries, including, for example, healthcare, military, paramilitary organizations, first responders, such as firemen and ambulatory personnel, emergency and surgical personnel, sports teams and many others. The following provides further description of certain embodiments of the invention. As described and claimed here, certain terms are defined and used interchangeably.

As used herein, "β-alanine", "beta-alanine", and "BA" are meant to represent the amino acid beta-alanine that is a free amino acid, or a salt of the free amino acid. As will be understood, these terms are to be used interchangeably except as otherwise specified herein. The use of these interchangeable terms does not encompass beta-alanine as a component of a dipeptide, oligopeptide, or polypeptide. Consequently, a human dietary supplement containing a dipeptide, oligopeptide, or polypeptide without any free amino acid beta-alanine, or salt thereof, would not be within the scope of the present invention. For example, a dietary supplement of carnosine, or the like, without any free amino acid beta-alanine, would not be within the scope of the present invention. If, however, a human dietary supplement comprises a dipeptide, oligopeptide, or polypeptide in combination with the free amino acid beta-alanine, or salt thereof, then such human dietary supplement would be within the scope of the present invention, provided the free amino acid beta-alanine, or salt thereof, is present in an effective amount as defined herein. Naturally, the ester and amide forms of the free amino acid beta-alanine, and their salts, could be used in a similar manner, although those forms are not in these originally submitted claims. Additionally, the use of these interchangeable terms in describing the human dietary supplement of the invention does not encompass beta-alanine from a natural or conventional food or food product unless otherwise specifically stated or claimed. Natural or conventional foods or food products include, but are not limited to, beef, pork, chicken, meat extract supplements, and predigested meat/protein supplements, and the various essences of meats. Under these definitions, the term "human dietary supplement" does not encompass, and does not mean, a natural or conventional food or food product, such as chicken meat, meat essences, chicken broth or meat flavoring.

As used herein, the term "human dietary supplement" is intended to mean a dietary supplement as defined under the Dietary Supplement Health and Education Act of 1994 ("DSHEA"). A human dietary supplement as used herein, also means a dietary supplement that is administered or taken by an individual more than once with the purpose of supplementing the diet to increase and/or maintain a component (*e.g.*, beta-alanine) of the supplement, or a substance comprising a component of the supplement (*e.g.*, carnosine) in the body at a higher level(s) than that naturally occurring through natural or conventional meals. Additionally, human dietary supplement further means an addition to the human diet in a pill, capsule, tablet, powder, or liquid form, which is not part of a natural or conventional food or food product, and which effectively increases the function of tissues when consumed.

As used herein, the term "period of time", "over time" or "duration of time" means more than a single dosing, taking or administration of the human dietary supplement. More specifically, these terms mean the human dietary supplement is taken one or more times per day over a period of seven or more days, wherein generally no two consecutive days pass without the dietary supplementation and the individual supplements the diet at least 3 or 4 days in any 7 day period, more preferably 4 or more days in any 7 day period, more preferably 5 or more days in any 7 day period, more preferably 6 or more days in any 7 day period, more preferably 7 consecutive days in any 7 day period. For example, the individual can take the dietary supplement every day, wherein the dietary supplement is provided over the course of the day or the individual may take a single dose of the dietary supplement for each day. The individual may also account for non-supplementation days as described above regarding days without supplementation. The period of time described herein can be continued for at least 7 days to about 240 days; preferably about 14 days to about 210 days; more preferably about 21 days to about 180 days; more preferably about 28 days to about 180 days; more preferably about 28 days to about 60 days; even more preferably about 30 days. It will be understood by those of skill in the art, that the period of time can be adjusted by the individual depending on the desired level of performance to be achieved and/or maintained.

As used herein, the term "effective amount" or "amount effective to" refers to an amount of the supplement required to achieve the increases or improvements sought and is an amount that is more than contained in the average diet. For example, omnivores consume about 50-300mg of carnosine per day and the cooking procedures used would lead to a beta-alanine amount lower than this. It will be understood by those skilled in the art that a one time, single dosing of beta-alanine is incapable of achieving an effective amount for the purposes of dietary supplementation with beta-alanine. Furthermore, it will be understood by those skilled in the art that administering a single dose followed with multiple consecutive days of non-dosing or non-supplementation will not achieve the effective amount as described in the invention.

As used herein, the terms "post-traumatic stress disorder", "PTSD", etc. refer to, but not limited to, a mental health condition that may be triggered by a traumatic event. The traumatic event may be experienced and/or witnessed. Symptoms may include, but are not limited to, recurrent, unwanted distressing memories of a traumatic event, reliving a traumatic event as if it were happening again (flashbacks), upsetting dreams about a traumatic event, severe emotional distress or physical reactions to something that brings to mind a traumatic event, trying to avoid thinking or talking about a traumatic event, avoiding places, activities or people that bring to mind a traumatic event, negative feelings about oneself or other people, inability to experience positive emotions, feeling emotionally numb, lack of interest in activities once enjoyed, hopelessness about the future, memory problems, such as not remembering important aspects of the traumatic event, difficulty maintaining close relationships, irritability, angry outbursts or aggressive behavior, always being on guard for danger, overwhelming guilt or shame, self-destructive behavior, such as drinking too much or driving too fast, trouble concentrating, trouble sleeping, being easily startled or frightened, and combinations thereof.

The invention provides an important understanding of how a nutrient or dietary supplement provided over a period of time can improve physiological responses related to post-traumatic stress disorder.

Forms and Formulations

Administration of the beta-alanine can be as the free amino acid beta-alanine, wherein the free amino acid is not part of a dipeptide, oligopeptide or polypeptide. The free amino acid can be a salt of beta-alanine. The free amino acid can be in a pill, tablet, capsule, granule or powder form. The free amino acid can be administered as part of a solid, liquid or semi-liquid. The free amino acid can be administered as part of a drink (*e.g.,* sports drink) or a food (*e.g.,* health bar).

The beta-alanine may also be administered in a sustained release formulation, wherein the free amino acid beta-alanine is not part of a dipeptide, oligopeptide, or polypeptide. The beta-alanine administered in a sustained release formulation may also be present as a salt of the beta-alanine. The sustained release formulation can be in a tablet, capsule, granule or powder form. The sustained release formulation can be administered as part of a solid, liquid or semi-liquid. The sustained release formulation of the free amino acid beta-alanine can be administered as part of a drink (*e.g.,* sports drink) or a food or food matrix (*e.g.,* health or energy bar or energy gel). It has been reported that some individuals may experience a slight flushing/tingling of the skin when taking β-alanineas a free amino acid. While this sensation may be uncomfortable, it typically lasts less than 60 minutes. The use of sustained-release forms of beta-alanine has been shown to inhibit, decrease and/or eliminate the flushing/tingling of the skin.

In various embodiments of the present invention, the human dietary supplement may be administered (*e.g.,* consumed or ingested) in combination with other ingredients. For example, the free amino acid beta-alanine, or a salt thereof, may be administered in combination with creatine, wherein the creatine is in the form of creatine-monohydrate or other acceptable forms of creatine. Creatine is desirable due to the enhanced ergogenic effect of the formulations of the current invention.

In another embodiment, the dietary supplement comprising the free beta-alanine can further comprise one or more carbohydrates, including simple carbohydrates, for example. Additionally, carbohydrates can include starch and/or sugars, *e.g.,* glucose, fructose, galactose, sucrose, and maltose. The sugars or other carbohydrates can be from various forms of honey, molasses, syrup (*e.g.,* corn syrup, glucose syrup), treacle or gels. It will be understood that the human dietary supplement of the invention may comprise one or more carbohydrates in combination with the other ingredients disclosed herein and as part of the forms and formulations defined by the present invention.

In addition, the human dietary supplements of the present invention may further comprise insulin, insulin mimics, and/or insulin-action modifiers. Insulin mimics include, but are not limited to, D-pinitol (3-O-methyl-chiroinositol), 4-hydroxy isoleucine, L783,281 (a demethyl-asterriquinone B-1 compound), alpha lipoic acid, R-alpha lipoic acid, guanidiniopropionic acid, vanadium compounds such as vanadyl sulfate or vanadium complexes such as peroxovanadium, and synthetic phosphoinositolglycans (PIG peptides). Insulin-action modifiers that enhance or inhibit the action of insulin in the body, can include, but are not limited to, sulphonylureas, thiazolidinediones, and biguanides. Additionally, the human dietary supplements may comprise insulin stimulating agents (*e.g.,* glucose).

In another embodiment, the dietary supplement comprising the free beta-alanine can further comprise one or more electrolytes and/or vitamins (*e.g.,* vitamins B6, B12, E, C, and thiamin, riboflavin, niacin, folic acid, biotin and pantothenic acid). In other embodiments, the human dietary supplement may comprise lipids, other amino acids, fiber, trace elements colorings, flavors, natural and/or artificial sweeteners, natural health improving substances, anti-oxidants, stabilizers, preservatives, and buffers.

In certain other embodiments, the human dietary supplement of the present invention may comprise other ingredients, for example, anti-oxidants, alpha-lipoic acid, tocotrienols, N-acetylcysteine, co-enzyme Q-10, extracts of rosemary such as carnosol, botanical anti-oxidants such as green tea polyphenols, grape seed extract, COX-1 type inhibitors such as resveratrol, ginkgo biloba, pterostilbene and garlic extracts. Other amino acids such as L-histidine, L-cysteine and/or L-citrulline may be added. In some embodiments, the present invention may comprise combination with an acetylcholine precursor such as choline chloride or phosphatidylcholine may be desirable, for example, to enhance vasodilation. The invention also provides for human dietary supplements comprising the free amino acid beta-alanine in combination with such other ingredients as minerals and trace elements in any type or form suitable for human consumption. It is convenient to provide calcium and potassium in the form of their gluconates, phosphates or hydrogen phosphates, and magnesium as the oxide or carbonate, chromium as chromium picolinate, selenium as sodium selenite or selenate, and zinc as zinc gluconate.

The ingredients, compounds and components disclosed herein as optionally being in the human dietary supplement comprising the free amino acid beta-alanine, may be in any combination as part of the human dietary supplement. This will be readily understood by those of skill in the field of dietary supplementation and exercise physiology.

Once the levels of beta-alanylhistidine have been increased by use of effective amounts of the human dietary supplement, otherwise known as a loading phase, the dosing can be adjusted to maintain the levels of beta-alanylhistidine necessary to improve physiological responses related to post-traumatic stress disorder for the purposes of this invention.

The forms and formulations, provided herein, can be those as described and provided for in U.S. Patent Nos. 5,965,596, 6,426,361, 7,825,084, 8,067,381, and 8,329, 207.

In one aspect, the dietary supplement is formulated for one or more servings that can be ingested one or more times per day to achieve an effective amount as required by the present invention. Thus, the total daily intake amount required to meet an effective amount of free beta-alanine, or a salt thereof, can be obtained through a single serving or through multiple smaller servings throughout the day that in total meet the required amount of free beta-alanine, or a salt thereof, to be an effective amount in a total daily intake of the dietary supplement. Therefore, a dietary supplement can be formulated with lower amounts of free beta-alanine, or a salt thereof, for the purpose of multiple servings in a day, wherein the total amount through multiple servings meets the desired total daily intake to be an effective amount as defined by the present invention.

The beta-alanine may be administered at a concentration of approximately 100g/kg of composition. In certain embodiments, the concentration may be approximately 70g/kg - approximately 130 g/kg, approximately 80 g/kg - approximately 120 g/kg, and/or approximately 90 g/kg - approximately 110 g/kg.

The beta-alanine may be provided as a mixture (composition) of free amino acid beta-alanine, or a salt thereof, and one or more other compounds. The one or more other compounds may be, but is not limited to, glucomannan. In certain embodiments, the mixture may be approximately 80% free beta-alanine, or a salt thereof, and approximately 20% glucomannan. In certain embodiments, the percentage of free beta-alanine, or a salt thereof, may be from approximately 50% - approximately 95%, approximately 60% - approximately 90%, and/or approximately 70% - approximately 85%.

The mixture may be provided as a solution. The solution may include approximately 1.0 - 1.2 grams of composition per approximately 100 ml water.

The total daily intake amount of the free amino acid beta-alanine, or a salt thereof, is in a range of about 0.3 grams (g) to about 16.0 g; preferably about 1.0 g to about 10.0 g; more preferably about 2.0 g to about 8.0 g; and even more preferably about 3.0 g to about 7.0 g. As described in the present invention, the total daily intake amount in these ranges can be achieved through a single serving formulation comprising the desired effective amount of free beta-alanine. Alternatively, the total daily intake amount in these ranges can be achieved through a formulation for multiple servings, each comprising an amount of the free beta-alanine that when totaled for the day will be within the desired range for a total daily intake delivering an effective amount as defined by the present invention.

Where the dietary supplement is formulated for multiple servings per day within the ranges described herein, it will be understood that there can be 2-12 servings or more, depending on the amounts of free beta-alanine, or salt thereof, in the formulated units. For example, a sustained release tablet comprising 2.0 g of free beta-alanine can be served 3 times per day for a total daily intake of 6.0 g of free beta-alanine. As another example, a formulation comprising 0.5 g of free beta-alanine can be taken 12 times throughout the day for a total daily intake of 6.0 g. This aspect of the present invention applies whether 12 tablets comprising 0.5 g of free beta-alanine are taken at 12 different times throughout the day or if 4 tablets are taken at 3 different times throughout the day. As will be understood in the present invention, it is the total daily intake of the free beta-alanine that must be an effective amount as defined by the present invention. Moreover, the effective amounts in the ranges provided herein account for non-supplementation days as defined by the present invention. Therefore, as long as the individual supplements his/her diet as described herein, the total daily intake of the dietary supplement accounts for non-supplementation days and achieves an effective amount as required over time.

These ranges for total daily intake of free beta-alanine can also account for the various body sizes. Therefore, it will be understood that individuals with smaller body types can take less or more depending on the desired performance levels to be achieved. Likewise, the ranges for total daily intake account for individuals with larger body types that might require a higher total daily intake to achieve the desired performance levels. Regardless of body type, the total daily intake of the effective amounts of free beta-alanine, or a salt thereof, in the dietary supplements of the present invention can account for adjustments in amounts based on the individual's body type requirements and desired performance levels.

It will also be understood that an effective amount being consumed, as defined herein, can be adjusted up or down as long as the total daily intake of free beta-alanine, or a salt thereof, is maintained within the ranges provided herein and meet the definition of effective amounts of the present invention. For example, an individual taking a dietary supplement of the present invention in a formulation delivering a total daily intake of 6.0 g or 6.4 g, can adjust the level of supplementation down to 1.6 g, 2.0 g, 3.0 g or 3.2 g of a total daily intake of the free amino acid beta-alanine, or a salt thereof. This is referred to as a maintenance phase. It will be understood by those of skill in the art that individuals may reach a desired level of performance through the dietary supplementation of the present invention and then the individual can opt to reduce the effective amount to a lower effective amount of the present invention to maintain the level of performance achieved. In a converse example, an individual taking a total daily intake of 3.0 g or 3.2 g of the free beta-alanine, or a salt thereof, as an effective amount, can increase the total daily intake of the free beta-alanine to any effective amount within the ranges described herein. For example, the individual could increase the total daily intake from 1.6 g, 2.0 g, 3.0 g or 3.2 g to a total daily intake of 6.0 g or 6.4 g, if a further increase in performance is desired. These examples of adjusting the total daily intake of the free beta-alanine described herein are intended as examples of how an individual can increase the level of performance or maintain an achieved level of performance.

It will also be understood from the present disclosure that an individual can cycle the intake of an effective amount of the free beta-alanine between higher and lower total daily intakes of an effective amount of beta-alanine. For example, an individual could take a total daily amount of 6.4 g of the free beta-alanine, or a salt thereof, as an effective amount for a period of 28 days, including non-supplementation days, followed by 28 days of taking 1.6 g of the free beta-alanine, or a salt thereof, as an effective amount, including non-supplementation days, followed by 28 days of taking 6.4 g of the free beta-alanine, or a salt thereof, as an effective amount, including non-supplementation days. It will also be understood that the time periods and total daily intake amounts given in the example of cycling can be adjusted based on the individual's body type requirements and desired performance levels.

As will be understood by one of skill in the art through the disclosure of the present invention, other ingredients, *e.g.*, creatine, other amino acids, and carbohydrates, can be present in the human dietary supplement in similar amounts as that described for the free amino acid beta-alanine, or salts thereof.

Example

In one embodiment, 30-days of β-alanine(BA) ingestion (100g·kg-1) was studied on the behavioral and neuroendocrine response in an animal model of post-traumatic stress disorder (PTSD). Animals were fed a normal diet with or without (PL) BA supplementation (100mg·kg-1) for 30-days. Following the supplementation period animals, were exposed to a predator-scent stress (PSS), which has been validated to use in an animal model stimulating PTSD-like behavior, or a sham (UNEX). Behaviors were evaluated with the elevated plus maze and acoustic startle response 7 days following exposure. Corticosterone concentrations (CS), an expression of brain-derived neurotrophic factor (BDNF), and brain carnosine concentrations were analyzed a day later. Animals in PSS+PL spent significantly less time in the open arms and in the number of entries in the EPM than PSS+BA, UNEX+BA, or UNEX+PL. Animals in PSS+BA had comparable scores to UNEX+BA. Anxiety index was higher ($p < 0.05$) in PSS+PL compared to PSS+BA or animals that were unexposed. ASR and freezing were greater ($p < 0.05$) in animals exposed to PSS compared to animals unexposed. CS expression was higher ($p < 0.05$) in animals exposed to PSS compared to unexposed animals. Brain carnosine concentrations in the hippocampus and other brain sections were significantly greater in animals supplemented with BA compared to PL. BDNF expression in the CA1 and DG subregions of the hippocampus was lower ($p < 0.05$) in animals exposed and fed a normal diet compared to animals exposed and supplemented with BA, or animals unexposed. As such, BA supplementation may provide an increase in brain carnosine concentrations, and a reduction in PTSD-like behavior, which may be mediated in part by maintaining BDNF expression in the hippocampus.

In conclusion, the results of this study indicate that 30-days of β-alanineingestion can improve physiological responses related to post-traumatic stress disorder.

**METHODS**

**Animals:**

Adult male Sprague-Dawley rats weighing 200-250 gm (n = 122) were habituated to housing conditions for at least seven days. All animals were housed four per cage in a vivarium with stable temperature and a reversed 12-h light/dark cycle, with unlimited access to food and water. β-alaninewas provided with glucomannan in a powder form in an 80:20 blend. Rats were provided with 100 mg of the powder per kg of body mass (a total of 30 mg of powder was dissolved in 25 ml of water). PL treated rats were provided with the vehicle (glucommanan) at the same relative dose. Animals were handled once daily. All testing was performed during the dark phase in dim red light conditions. This study was performed according to the principles and guidelines of the National Institute of Health Guide for the Care and Use of Laboratory Animals. All treatment and testing procedures were approved by the Animal Care Committee of the Ben-Gurion University of the Negev, Israel.

**Experimental Design**

Rats were randomly assigned to one of four treatment groups (n = 30 or 31 per group):

1) Unexposed and vehicle-treated group (UNEXP+PL): rats were fed regular food and water for 30 days and were exposed to fresh, unused litter for 15 min.

2) Unexposed and treated with β-alanine(UNEXP+BA): rats were provided β-alanine in their water and were exposed to fresh, unused litter for 15 min.

3) Exposed and vehicle-treated (EXP+PL): rats were fed regular food and water for 30 days and were exposed to PSS for 15 min.

4) Exposed and treated with β-alanine(EXP+BA): rats were provided β-alanine in their water exposed to PSS.

Following the 7-day acclimation period in which all rats received a normal powder diet, they were randomized into the four groups. Following 30-days of either normal diet or β-alaninesupplemented diet the rats were exposed to either the PSS or sham protocol. All behavioral tests were conducted 7 days following the PSS or sham protocol, and the rats were then sacrificed 24 hours later and brains were removed. Diets were maintained until the end of the study. The validity of this model has been demonstrated in several studies (Cohen et al., 2004; 2012b; Kozlovsky et al., 2007).

**Predator-Scent Stress (PSS)**

Following the 30-day supplementation regimen, animals were exposed to the PSS protocol. The PSS protocol consisted of placing the experimental animal on well-soiled cat litter (in use by the cat for 2 days, sifted for stools) for 10 minutes in a closed environment. Control animals were exposed to fresh, unused litter for the same amount of time. The situational reminder consisted of placing animals on fresh, unused cat litter for 15 minutes.

**Assessment Schedule**

Behavioral responses were assessed in the elevated plus-maze, acoustic startle response and contextual freezing. All results were recorded and analyzed using an EthoVision automated tracking system (Noldus Information Technology, The Netherlands). Performance in the elevated plus-maze and acoustic startle response occurred 7-days following the initial exposure to the PSS. The contextual freezing measures were performed on days 8 following initial exposure. The delay in performing these measures from the PSS is based upon findings that extreme behavioral changes, which remain constant after 7 days of exposure and represent 'chronic symptoms' (Cohen et al. 2004) which persist over a prolonged duration (Cohen and Zohar, 2004; Cohen et al., 2004). Following behavioral assessments, all animals were sacrificed and the brains were removed for analysis.

**Behavioral Measures**

*Elevated Plus-Maze (EPM)*

Behavioral assessments performed in the EPM have previously been described (Cohen H et al., 2003; 2012). The EPM is a plus-shaped platform with two opposing open and two opposing closed arms (open only towards the central platform and surrounded by 14-cm high opaque walls on three sides). Rats were placed on the central platform facing an open arm and allowed to explore the maze for 5 min. Each session was videotaped and subsequently scored by an independent observer. Arm entry was defined as entering an arm with all four paws. Behaviors assessed were: time spent (duration) in open and closed arms and on the central platform; number of open and closed arm entries; and total exploration (entries into all arms). Total exploration was calculated as the number of entries into any arm of the maze in order to distinguish between impaired exploratory behavior, exploration limited to closed arms (avoidance) and free exploration. "Anxiety Index", an index that integrates the EPM behavioral measures, was calculated as follows:

$$Anxiety \quad Index = 1 - \left[ \frac{\left( \frac{time \quad spent \quad in \quad the \quad open \quad arms}{total \quad time \quad on \quad the \quad maze} \right) + \left( \frac{number \quad of \quad entries \quad to \quad the \quad open \quad arms}{total \quad \exp loration \quad on \quad the \quad maze} \right)}{2} \right]$$

Anxiety Index values range from 0-1 where an increase in the index expresses increased anxiety-like behavior

*Acoustic Startle Response*

Startle response was measured using two ventilated startle chambers (SR-LAB system, San Diego Instruments, San Diego, CA). The SR-LAB calibration unit was used routinely to ensure consistent stabilimeter sensitivity between test chambers and over time. Each Plexiglas cylinder rests on a platform inside a sound-proofed, ventilated chamber. Movement inside the tube is detected by a piezoelectric accelerometer below the frame. Sound levels within each test chamber are measured routinely using a sound level meter to ensure consistent presentation. Each test session started with a 5-min acclimatization period to background white noise of 68 dB, following by 30 acoustic startle trial stimuli in six blocks (110 dB white noise of 40 ms duration with 30 or 45 sec inter-trial interval). Behavioral assessment consisted of mean startle amplitude (averaged over all 30 trials) and percent of startle habituation to repeated presentation of the acoustic pulse. Percent habituation -- the percent change between the response to the first block of sound stimuli and the last - was calculated as follows:

$$Percent \quad Habituatio\, n = 100 \times \left[ \frac{(average \quad startle \quad amplitude \quad in \quad Block \quad 1) - (average \quad startle \quad amplitude \quad in \quad Block \quad 6)}{(average \quad startle \quad amplitude \quad in \quad Block \quad 1)} \right]$$

*Contextual Freezing Measurement*

Freezing behavior was scored during the situational reminder/cue exposure and was defined as an absence of all movement (except for respiration) (Kim et al. 1992). Total cumulative freezing time (total seconds spent freezing during each assessment period) was measured and calculated as a percentage of total time. Freezing behavior was recorded using an overhead video camera and scored for immobility using the recorded images. The videotape and the recorded images were both scored by a trained observer unaware of the treatment conditions.

**Blood Sampling**

Twenty rats from each group were decapitated with a guillotine. Care was taken to minimize situational stress: the area was thoroughly cleaned between each sacrifice and bodies were removed. Trunk blood was collected and left at room temperature for 2 hr and then centrifuged (1000g for 10 min at 4°C) with a Hermle centrifuge. Serum (approximately 1 ml from each rat) was collected and stored at -80°C until the analysis was performed.

**Measurement of Serum Corticosterone**

Serum corticosterone was measured with ELISA Test Kit (Endocrine Technologies Inc. Newark, CA) according to the instructions of the manufacturer. Plates were read at 450 nm. All reactions were determined in duplicate. Inter-assay variation was 6.1% and intra-assay variation 5.9%. All samples were measured in duplicate. The lowest level detectable in this assay is 0.1 ng/mL of serum.

**Brain BDNF levels:**

**Tissue Preparation**

Twenty-four hours following the behavioral tests, 10 animals from each group were deeply anesthetized (ketamine and xylazine mixture) and perfused transcardially with cold 0.9% physiological saline followed by 4% paraformaldehyde (Sigma-Aldrich) in 0.1 M phosphate buffer (pH 7.4). Brains were quickly removed, postfixed in the same fixative for 12 h at 4 °C, and cryoprotected overnight in 30% sucrose in 0.1 M phosphate buffer at 4° C. Brains were frozen on dry ice and stored at -80 °C. Serial coronal sections (10 $\mu$m) at the level of dorsal hippocampus were collected for each animal, using a cryostat (Leica CM 1850) and mounted on coated slides.

**Immunofluorescence:**

Sliced sections were air dried and incubated in frozen methanol (2 min) and in 4% Para-formal-aldehyde (4 min). After three washes in phosphate buffer saline (PBS) containing Tween 20 (PBS/T) (Sigma-Aldrich), the sections were incubated for 60 min in a blocking solution in (normal goat or horse serum in PBS) and then overnight at 4 °C with the primary antibodies against BDNF (1:250 each; Abcam). After three washes in PBS/T, sections were incubated in DyLight-488 in PBS containing 2% normal serum for 2 h. Sections were washed, mounted with mounting medium (Vectrastain Vector laboratories, USA). Control staining was performed in the absence of the primary antibodies. Additionally, secondary fluorescent labels were swapped to check cross-reactivity and sections were incubated without any primary antibodies to check for any non-specific binding of the secondary antibodies.

**Quantification:**

A computer-assisted image analysis system (Leica Application Suite V3.6, Leica, Germany) was used for quantitative analysis of the immunostaining and $50\times$ objective lens were employed to assess the number of BDNF-IR positive cells in the hippocampus, divided into three (counted separately) areas: CA1 subfield, CA3 subfield and dentate gyrus (DG). The regions of interest were outlined and computer-aided estimation was used to calculate the number of BDNF-IR cells in the pyramidal layer of CA1 and CA3, and in the granular layer of the DG. Seven representative sections of the hippocampus were chosen (between Bregma - 2.30 and Bregma -3.60) from each animal, from each group. The sections were analyzed by two observers blinded to the treatment protocol. Standard technique was used to estimate the number of BDNF cells profiles per unit area for each investigated hippocampal structure.

**Measurement of Brain Carnosine Concentrations**

Carnosine concentrations in brain homogenates were determined by LC-MS-MS analysis.
Brains were partially thawed on ice and six brain regions were sampled: cerebral cortex, hypothalamus, hippocampus, amygdala, olfactory bulb, and thalamus. Each sample was weighed and transferred into individual vials for further homogenization.
Brain samples were homogenized in saline (0.9% NaCl; 100 mg/1 ml) supplemented with 1 mM EDTA, using Polytron Homogenizer. Homogenates were stored at -20° $\pm$ 5°C prior to LC/MS/MS analysis. L-Carnosine purchased from Sigma was used to prepare standards for the calibration curve (in ppb). Samples were diluted x5 prior to analysis. The concentration read from the standard curve was multiplied by the dilution factor and converted to ppm units.

**Statistical analyses:**

The statistical analyses were performed using a one-way analysis of variance (ANOVA). In the event of a significant F ratio, LSD post-hoc analysis was used for pairwise comparisons. All data are reported as mean $\pm$ SD. Pearson's product-moment correlation was used to determine selected bivariate correlations. An alpha level of $p < 0.05$ was used to determine statistical significance. Data were analyzed using SPSS v22 software (SPSS Inc., Chicago, IL).

**RESULTS:**

***Elevated Plus-Maze:***

Comparisons between the groups for time spent in open arms indicated a significant effect ($F (3, 118) = 7.06$, $p < 0.001$). Post-hoc analyses indicated that animals that consumed a normal diet and were exposed to PSS spent significantly less time in the open arms than all other treatment groups (p's ranging from < 0.001 - 0.012). No other between group differences were noted in time spent in open arms (*See* Fig. 1A). Comparisons of open arm entries (*See* Fig. 1B) also showed a significant interaction between groups ($F (3, 118) = 9.55$, $p < 0.001$). Normal diet fed animals that were exposed to PSS exhibited significantly (p's < 0.001) fewer entries than all other treatment groups. (p's < 0.001). No other group differences were noted in open arm entries.
Analysis of the total activity on the EPM of all groups (*See* Fig. 1C) revealed no significant differences between the groups ($F (3, 118) = 0.925$, $p = 0.431$). Based upon the integrated behavioral measures a significant difference in the anxiety-index was noted between the groups (*See* Fig. 1D) ($F (3, 118) = 8.05$, $p < 0.001$). Animals fed a normal diet and exposed to PSS exhibited significantly greater anxiety (p's ranging from < 0.001 - 0.002) than other treatment groups (*See* Fig. 1D). No other between group effects were noted.

*Acute Startle Response and Startle Habituation:*

Comparisons between the groups in acoustic startle amplitude revealed a significant group difference (F (3, 118) = 14.02, p < 0.001) *(See* Fig. 2A). Startle amplitude was significantly greater for animals that were exposed to PSS and fed a normal diet compared to unexposed animals, regardless of diet (p's < 0.001), as well as for animals exposed to PSS and fed BA compared to unexposed animals fed a normal diet (p < 0.001) or supplemented with BA (p = 0.0001). In addition, animals that were exposed to PSS and whose diets were supplemented with BA tended to have a lower startle amplitude than exposed animals that were not fed BA (p = 0.085). A significant difference was also noted in startle habituation (F (3, 118) = 9.11, p < 0.001) *(See* Fig. 2B). Animals exposed to PSS regardless of diet had a significantly lower habituation score than all other groups (p's < 0.001). No other significant differences between groups were noted.

*Effect of cue-exposure on freezing behavior at Day 8:*

Fig. 3 depicts the freezing behavior upon cue-exposure. A significant difference between the groups was seen in freezing behavior (F (3,118) = 9.342, p < 0.001). Animals exposed to PSS and fed a normal diet had significantly greater immobility upon cue than animals that were unexposed and fed a normal diet (p < 0.001) or fed a diet supplemented with BA (p < 0.001). Animals exposed to PSS and supplemented with BA were also observed to have significantly greater immobility upon cue than animals that were unexposed and fed a normal diet (p = 0.01) or fed a diet supplemented with BA (p = 0.003). Animals exposed to PSS and fed BA froze approximately 6% less (40.9% versus 34.8%) less than animals exposed to PSS and fed a normal diet. However, this difference was not statistically different (p = 0.148).

*Corticosterone concentrations at Day 8 post-PSS exposure:*

A significant difference between the groups (Fig. 4) was observed in corticosterone concentrations (F (3, 76) = 13.883, p < 0.001). Corticosterone concentrations in animals exposed to PSS and fed a normal diet were significantly greater than animals not exposed and fed a similar diet (p < 0.001) or supplemented with BA (p < 0.001). In addition, animals that were exposed to PSS and supplemented with BA were also observed to have significantly higher corticosterone concentrations than animals that were unexposed and fed a normal diet (p < 0.001) or were unexposed and supplemented with BA (p < 0.001). No significant differences (p = 0.837) were noted between exposed rats fed a normal diet compared to exposed rats supplemented with BA.

*BDNF expression at Day 8 post-PSS exposure:*

Comparisons between the groups in BDNF expression in the CA1, CA3 and DG subregions can be observed in Figs. 5A - 5C, respectively. Significant differences were noted in the CA1 (F (3, 36) = 23.2, p < 0.001) and DG subregions (F (1, 36) = 4.126, p = 0.013). No differences were observed between the groups in the CA3 subregion (F (1, 36) = 0.918, p = 0.442). BDNF expression in the CA1 and DG subregions in the animals that were exposed to PSS and fed a normal diet were significantly lower than all other groups (p's ranging from < 0.001 - 0.011). In addition, BDNF expression in animals exposed to PSS and supplemented with BA were significantly lower than unexposed animals (r's ranging from 0.009 - 0.024), but significantly greater than exposed rats fed a normal diet in the CA1 (p < 0.001) only. In the DG subregion, BDNF expression in animals exposed to PSS but supplemented with BA were not statistically different from animals not exposed and either fed a normal diet (p = 0.765) or supplemented with BA (p = 0.818), however the expression of BDNF in these animals exposed and fed BA was greater (p = 0.011) than animals exposed and fed a normal diet. No other differences were noted.

*Brain Carnosine Concentrations at Day 8 post-PSS exposure:*

The concentration of carnosine in various areas of the brain can be observed in Table 1. Significant differences were noted in the hippocampus (F (3, 36) = 5.818, p = 0.002), cerebral cortex (F (1, 36) = 6.659, p = 0.001, hypothalamus (F (3, 36) = 5.348, p = 0.004), amygdala (F (3, 36) = 3.825, p = 0.018), and thalamus (F (3, 36) = 5.162 p = 0.005). No significant differences were noted in the olfactory bulb (F (3, 36) = 2.051, p = 0.124). In the hippocampus, cortex, hypothalamus, amygdala, and thalamus animals that ingested BA and were exposed to PSS had significantly greater carnosine concentrations (p's ranging from < 0.001 - 0.011) than animals who were fed a normal diet, regardless of exposure. Animals that were not exposed to PSS and supplemented with BA had a significantly greater carnosine concentration in the hippocampus (p = 0.030) and thalamus (p = 0.042) compared to animals not exposed and fed a normal diet. Additional comparisons between animals that were not exposed to PSS and supplemented with BA appeared to result in only strong trends (p's ranging from 0.051 - 0.089) when compared to animals fed a normal diet, regardless of exposure, in the cerebral cortex, hypothalamus and amygdala. No differences were noted in comparisons between animals unexposed and fed BA compared to animals exposed and fed a normal diet in the hypothalamus (p = 0.113) and amygdala (p = 0.115) only.

**Table 1: Brain Carnosine Concentrations (mg·L$^{-1}$)**

|  | Hippocampus | Cortex | Hypothalamus | Amygdala | Olfactory bulb | Thalamus |
|---|---|---|---|---|---|---|
| UNEX + PL | $0.71 \pm 0.59$ | $0.76 \pm 0.79$ | $0.64 \pm 0.64$ | $0.30 \pm 0.34$ | $11,011 \pm 3,910$ | $0.77 \pm 0.46$ |
| UNEX + BA | $3.75 \pm 3.98$ **a** | $3.78 \pm 4.47$ **a** | $2.60 \pm 2.85$ | $2.34 \pm 3.04$ | $8,309 \pm 6,887$ | $3.99 \pm 5.10$ **a** |
| PSS + PL | $1.12 \pm 0.90$ | $0.96 \pm 0.43$ | $0.78 \pm 0.46$ | $0.55 \pm 0.50$ | $5,744 \pm 6,491$ | $1.16 \pm 0.70$ |
| PSS + BA | $5.58 \pm 4.39$ **a, c** | $6.55 \pm 4.90$ **a, c** | $4.54 \pm 4.04$ **a, c** | $3.52 \pm 3.84$ **a, c** | $13,109 \pm 9,773$ | $5.97 \pm 4.48$ **a, c** |

a = significantly different than UNEX + PL;
c = significantly different than PSS + PL;
UNEX + PL = animals that were not exposed to the predator scent stress and fed a normal diet;
UNEX + BA = animals that were not exposed to the predator scent stress and supplemented with β-alanine;
PSS + PL = animals that were exposed to the predator scent stress and fed a normal diet;
PSS + BA = animals that were exposed to the predator scent stress and supplemented with β-alanine.

All data reported as mean $\pm$ SD.

**DISCUSSION**

The results of this study indicated that 30-days of β-alanineingestion in rats was effective in attenuating some of the behaviors tested and associated with exposure to PSS. Rats fed a normal diet and exposed to PSS were observed to be significantly less active when placed in the elevated maze, and had a greater anxiety level compared to animals that were either unexposed, or animals that were exposed and supplemented with BA. Animals that were exposed but supplemented with β-alanine, saw a 19% lower startle response (p = 0.085) and a 15% lower freezing response (p = 0.148) than animals exposed and which were not supplemented.

The animal model of PTSD used in this study has been previously established as a valid and effective method of examining biomolecular and physiological parameters of specific response patterns of stress. This model has also been established as an effective approach to determine the effects of various interventions on the behavioral response to stress. Differences in the startle amplitude response (2.5 - 2.2 fold higher) seen between PSS+PL versus UNEX+PL or UNEX+BA, respectively, was consistent with the 2.5-fold increase in previous reports. Exposure to PSS in this study also resulted in a 30% elevation in anxiety index and a nearly 2-fold increase in the freezing response compared to animals unexposed.

Exposure to PSS was also accompanied by significant elevations in plasma corticosterone concentrations 7-days following the stress. This was consistent with other studies using the same stress model. Elevations in glucocorticoids are associated with exposure to stress, but recent evidence suggests that elevations in glucocorticoids may play a role in enhancing versus impairing recovery. Previous research has indicated that a blunted hypothalamic-pituitary adrenal axis response to an acute stress may increase the susceptibility for PTSD-like symptoms, which can be reversed with treatment of high-dose cortisone administration. Glucocorticoid concentrations, however, appear to demonstrate an inverted U effect. Although low concentrations may be a predictor for PTSD, high concentrations have also been suggested to be associated with morphological changes of neurons that also contribute to a negative behavioral response seen during stress. The hippocampus is sensitive to both acute and chronic stress. A chronic stress response may elevate glucocorticoid expression in the brain causing the neurons in the hippocampus to undergo reversible remodeling especially in the DG-CA3 regions. These changes often involve dendritic atrophy, which may result in diminished function in hippocampal-dependent memory tasks. In contrast, BDNF is reported to have the opposite effect on neuroplasticity by enhancing neurogenesis and dendritic remodeling. Elevations in corticosterone concentrations observed in both groups of rats exposed to PSS in this study were likely indicative of the stress experienced by those animals. The animals that were supplemented with BA, however, appeared to recover or demonstrated a greater resiliency to stress in regards to their levels of anxiety and performance in the EPM. This may have been a function of both elevations in brain carnosine levels and an increased expression of BDNF. This is supported by the association seen in the increased expression of BDNF in the CA1 and DG subregions observed in this study with a reduction in the startle response (r = -0.341, p = 0.031 and r = - 0.523. p = 0.001, respectively) and freezing response (r = -0.572, p < 0.001 and r = -0.335. p = 0.035, respectively) of the animals examined.

Rats supplemented with β-alanine in this study experienced significant elevations in carnosine concentrations in nearly all segments of the brain. Supplementation with β-alanine did not appear to have any influence on carnosine concentrations in the olfactory bulb. This was likely related to the high concentration of carnosine generally seen in the olfactory bulb of most mammalian species. However, differences in carnosine concentrations within the other brain regions supports previous studies that showed β-alanine supplemented diets can increase brain carnosine concentrations in mice and chickens. Brain carnosine may attenuate depression-like behavior by reducing brain metabolites of norepinephrine, or through stimulation of histaminergic neurons through one of its constituents; histidine. Others have suggested that carnosine might influence brain antioxidant activity, while others have reported that elevations in brain carnosine concentrations in the hypothalamus and cerebral cortex are associated with increases in the concentration of BDNF in the hippocampus of mice. In the present study elevations in brain carnosine concentrations in the PSS + BA group were associated with maintaining expression of BDNF in the CA1 and DG subregions of the hippocampus compared to PSS + PL rats. Elevations in carnosine concentrations in the hippocampus, cortex hypothalamus, amygdala and thalamus were inversely associated with anxiety index (r's ranging from -0.471 - -0.550, p's < 0.002) and positively associated with improved time spent in the open arms (r's ranging from 0.453 - 0.521, p's < 0.003).

The significant decrease in BDNF expression observed in rodents exposed to PSS and fed a normal diet is consistent with previous studies reporting an attenuation of BDNF expression and PTSD-like behavioral stress response. Although β-alanine ingestion and subsequent carnosine synthesis in the brain did not appear to increase BDNF expression in unexposed rats, it did appear to maintain BDNF expression in those rats that were exposed to PSS. Similar expression of BDNF expression in both UNEX+BA and UNEX+PL suggested that elevations in brain carnosine levels were not directly responsible for altering BDNF expression. These results on the surface may appear to contrast with studies that showed significant elevations in BDNF concentrations in hippocampus homogenates following β-alanine ingestion. Those investigators did not, however, use a non-stressed group as a control. The mechanism of elevated brain carnosine and maintenance of BDNF expression in the hippocampus is not well-understood, but it may be related to its role as a neural protectant through its action as an antioxidant. Oxidative stress and inflammation in the brain have been demonstrated to cause the development and further exacerbation of PTSD. Thus, carnosine's role as an antioxidant may prevent the neurodegeneration associated with elevated glucocorticoids and indirectly preserving BDNF expression.

In summary, the results of this study indicate that 30-days of β-alanine supplementation appears to promote resiliency and/or recovery from PSS. The protective effects associated with elevations in brain carnosine may be related to a protection of BDNF expression in the hippocampus. β-alanine may be used as a dietary supplement for the treatment or prevention of PTSD.

**Claims**

1. A human dietary supplement comprising a free amino acid beta-alanine, or a salt thereof, for use in therapeutically improving physiological responses related to post-traumatic stress disorder.

2. The dietary supplement for use of claim 1, wherein the physiological responses are selected from the group consisting of: unwanted distressing memories of a traumatic event, reliving a traumatic event as if it were happening again (flashbacks), upsetting dreams about a traumatic event, severe emotional distress or physical reactions to something that brings to mind a traumatic event, trying to avoid thinking or talking about a traumatic event, avoiding places, activities or people that bring to mind a traumatic event, negative feelings about oneself or other people, inability to experience positive emotions, feeling emotionally numb, lack of interest in activities once enjoyed, hopelessness about the future, memory problems, difficulty maintaining close relationships, irritability, angry outbursts or aggressive behavior, always being on guard for danger, overwhelming guilt or shame, self-destructive behavior, trouble concentrating, trouble sleeping, being easily startled or frightened, and combinations thereof.

3. The dietary supplement for use of claim 1, wherein the human dietary supplement is administered one or more times per day for multiple days.

4. The dietary supplement for use of claim 3, wherein the effective amount is at least about 3.0 g of the free amino acid beta-alanine, or salt thereof.

5. The dietary supplement for use of claim 3, wherein the multiple days is at least a fourteen day period.

6. The dietary supplement for use of claim 3, wherein the multiple days allows for non-supplementation days.

7. The dietary supplement for use of claim 6, wherein the non-supplementation is no more than one consecutive day and no more than two days in a seven day period.

8. The dietary supplement for use of claim 1, wherein the human dietary supplement is provided with a form of creatine, an insulin stimulator, a carbohydrate, one or more vitamins or one or more other proteins and amino acids.

9. The dietary supplement for use of claim 1, wherein the dietary supplement is administered prior to a user experiencing symptoms related to PTSD.

10. The dietary supplement for use of claim 1, wherein the dietary supplement is administered during a time when a user experiences symptoms related to PTSD.

11. The dietary supplement for use of claim 1, wherein the dietary supplement is administered after a user experiences symptoms related to PTSD.

12. The dietary supplement for use of claim 1, wherein the dietary supplement is administered prior to a user being diagnosed with post-traumatic stress disorder.

13. The dietary supplement for use of claim 1, wherein the dietary supplement is administered during a time when a user is diagnosed with post-traumatic stress disorder.

14. The dietary supplement for use of claim 1, wherein the dietary supplement is administered after a user is diagnosed with post-traumatic stress disorder.

15. The dietary supplement for use of claim 1, wherein the use further comprises the step of adjusting an effective amount of the dietary supplement once the improvement in physiological responses related to post-traumatic stress disorder are achieved to allow for a maintenance phase.

**Patentansprüche**

1. Ein Nahrungsergänzungsmittel für Menschen, das eine freie Aminosäure beta-Alanin oder ein Salz davon beinhaltet, zur Verwendung bei der therapeutischen Verbesserung physiologischer Reaktionen in Verbindung mit posttraumatischer Belastungsstörung.

2. Nahrungsergänzungsmittel zur Verwendung nach Anspruch 1, wobei die physiologischen Reaktionen aus der Gruppe ausgewählt sind, die aus den Folgenden besteht: unerwünschte schmerzliche Erinnerungen an ein traumatisches Ereignis, erneutes Durchleben eines traumatischen Ereignisses, als ob es erneut geschähe (Flashbacks), beunruhigende Träume über ein traumatisches Ereignis, schweres emotionales Leiden oder körperliche Reaktionen auf etwas, das an ein traumatisches Ereignis erinnert, Versuchen, das Denken an oder Reden über ein traumatisches Ereignis zu vermeiden, Vermeiden von Orten, Aktivitäten oder Menschen, die an ein traumatisches Ereignis erinnern, negative Gefühle über sich selbst oder andere Menschen, Unfähigkeit zum Erleben positiver Emotionen, Gefühl der emotionalen Taubheit, mangelndes Interesse an Tätigkeiten, die früher Freude gemacht haben, Hoffnungslosigkeit über die Zukunft, Gedächtnisprobleme, Schwierigkeiten beim Aufrechterhalten enger Beziehungen, Reizbarkeit, Wutausbrüche oder aggressives Verhalten, ständig auf der Hut vor Gefahren sein, überwältigendes Schuld- oder Schamgefühl, selbstzerstörerisches Verhalten, Konzentrationsprobleme, Schlafprobleme, Schreckhaftigkeit oder Furchtsamkeit und Kombinationen davon.

3. Nahrungsergänzungsmittel zur Verwendung nach Anspruch 1, wobei das Nahrungsergänzungsmittel für Menschen einmal oder mehrmals täglich über mehrere Tage hinweg verabreicht wird.

4. Nahrungsergänzungsmittel zur Verwendung nach Anspruch 3, wobei die wirksame Menge mindestens etwa 3,0 g der freien Aminosäure beta-Alanin oder eines Salzes davon beträgt.

5. Nahrungsergänzungsmittel zur Verwendung nach Anspruch 3, wobei die mehreren Tage ein mindestens vierzehntägiger Zeitraum sind.

**6.** Nahrungsergänzungsmittel zur Verwendung nach Anspruch 3, wobei bei den mehreren Tagen nahrungsergänzungsfreie Tage zulässig sind.

**7.** Nahrungsergänzungsmittel zur Verwendung nach Anspruch 6, wobei die nahrungsergänzungsfreie Zeit nicht mehr als ein aufeinanderfolgender Tag und nicht mehr als zwei Tage in einem siebentägigen Zeitraum ist.

**8.** Nahrungsergänzungsmittel zur Verwendung nach Anspruch 1, wobei das Nahrungsergänzungsmittel für Menschen mit einer Form von Kreatin, einem Insulinstimulator, einem Kohlenhydrat, einem oder mehreren Vitaminen oder einem oder mehreren anderen Proteinen und Aminosäuren bereitgestellt wird.

**9.** Nahrungsergänzungsmittel zur Verwendung nach Anspruch 1, wobei das Nahrungsergänzungsmittel vor dem Auftreten von Symptomen in Verbindung mit PTSD bei einem Anwender verabreicht wird.

**10.** Nahrungsergänzungsmittel zur Verwendung nach Anspruch 1, wobei das Nahrungsergänzungsmittel während einer Zeit, in der Symptome in Verbindung mit PTSD bei einem Anwender auftreten, verabreicht wird.

**11.** Nahrungsergänzungsmittel zur Verwendung nach Anspruch 1, wobei das Nahrungsergänzungsmittel vor dem Auftreten von Symptomen in Verbindung mit PTSD bei einem Anwender verabreicht wird.

**12.** Nahrungsergänzungsmittel zur Verwendung nach Anspruch 1, wobei das Nahrungsergänzungsmittel vor der Diagnosestellung der posttraumatischen Belastungsstörung bei einem Anwender verabreicht wird.

**13.** Nahrungsergänzungsmittel zur Verwendung nach Anspruch 1, wobei das Nahrungsergänzungsmittel während einer Zeit, in der die Diagnose der posttraumatischen Belastungsstörung bei einem Anwender gestellt wird, verabreicht wird.

**14.** Nahrungsergänzungsmittel zur Verwendung nach Anspruch 1, wobei das Nahrungsergänzungsmittel nach der Diagnosestellung der posttraumatischen Belastungsstörung bei einem Anwender verabreicht wird.

**15.** Nahrungsergänzungsmittel zur Verwendung nach Anspruch 1, wobei die Verwendung ferner den Schritt des Anpassens einer wirksamen Menge des Nahrungsergänzungsmittels beinhaltet, sobald die Verbesserung der physiologischen Reaktionen in Verbindung mit posttraumatischer Belastungsstörung erzielt worden ist, um eine Erhaltungsphase zu erlauben.

**Revendications**

**1.** Complément alimentaire humain comprenant un acide aminé libre bêta-alanine, ou un sel de celui-ci, destiné à une utilisation dans l'amélioration thérapeutique de réponses physiologiques associées à un trouble de stress posttraumatique.

**2.** Complément alimentaire destiné à une utilisation selon la revendication 1, les réponses physiologiques étant sélectionnées dans le groupe constitué de souvenirs pénibles et indésirables d'un événement traumatisant, du fait de revivre un événement traumatisant comme s'il se produisait à nouveau (flashbacks), de rêves bouleversants concernant un événement traumatisant, d'une détresse émotionnelle sévère ou de réactions physiques à quelque chose qui évoque un événement traumatisant, du fait d'essayer d'éviter de penser à ou de parler d'un événement traumatisant, du fait d'éviter les lieux, les activités ou les personnes qui font venir à l'esprit un événement traumatisant, de sentiments négatifs sur soi-même ou d'autres personnes, d'une incapacité à éprouver des émotions positives, d'un sentiment d'insensibilité émotionnelle, d'un manque d'intérêt pour des activités autrefois appréciées, d'un désespoir quant on pense à l'avenir, de problèmes de mémoire, d'une difficulté à entretenir de proches relations, d'une irritabilité, d'accès de colère ou d'un comportement agressif, du fait de toujours être sur ses gardes en guettant un danger, d'un sentiment écrasant de culpabilité ou de honte, d'un comportement autodestructeur, de troubles de la concentration, de troubles du sommeil, du fait d'être facilement surpris ou effrayé, et de combinaisons de ceux-ci.

**3.** Complément alimentaire destiné à une utilisation selon la revendication 1, le complément alimentaire humain étant administré une ou plusieurs fois par jour pendant plusieurs jours.

**4.** Complément alimentaire destiné à une utilisation selon la revendication 3, la quantité efficace étant d'au moins environ 3,0 g de l'acide aminé libre bêta-alanine, ou d'un sel de celui-ci.

5. Complément alimentaire destiné à une utilisation selon la revendication 3, lesdits plusieurs jours étant au moins une période de quatorze jours.

6. Complément alimentaire destiné à une utilisation selon la revendication 3, lesdits plusieurs jours permettant des jours de non-supplémentation.

7. Complément alimentaire destiné à une utilisation selon la revendication 6, la non-supplémentation ne durant pas plus d'un jour consécutivement et pas plus de deux jours au cours d'une période de sept jours.

8. Complément alimentaire destiné à une utilisation selon la revendication 1, le complément alimentaire humain comprenant une forme de créatine, un stimulateur d'insuline, un glucide, une ou plusieurs vitamines ou un(e) ou plusieurs autres protéines et acides aminés.

9. Complément alimentaire destiné à une utilisation selon la revendication 1, le complément alimentaire étant administré avant qu'un utilisateur n'éprouve des symptômes associés à un TSPT.

10. Complément alimentaire destiné à une utilisation selon la revendication 1, le complément alimentaire étant administré durant une période où un utilisateur éprouve des symptômes associés à un TSPT.

11. Complément alimentaire destiné à une utilisation selon la revendication 1, le complément alimentaire étant administré après qu'un utilisateur éprouve des symptômes associés à un TSPT.

12. Complément alimentaire destiné à une utilisation selon la revendication 1, le complément alimentaire étant administré avant qu'un utilisateur ne reçoive un diagnostic de trouble de stress post-traumatique.

13. Complément alimentaire destiné à une utilisation selon la revendication 1, le complément alimentaire étant administré durant une période où un utilisateur reçoit un diagnostic de trouble de stress post-traumatique.

14. Complément alimentaire destiné à une utilisation selon la revendication 1, le complément alimentaire étant administré après qu'un utilisateur a reçu un diagnostic de trouble de stress post-traumatique.

15. Complément alimentaire destiné à une utilisation selon la revendication 1, l'utilisation comprenant en outre l'étape d'ajustement d'une quantité efficace du complément alimentaire une fois que l'amélioration des réponses physiologiques associées à un trouble de stress post-traumatique a été obtenue, pour permettre une phase d'entretien.

FIG. 1A

FIG. 1B

FIG. 1C

FIG. 1D

FIG. 2A

FIG. 2B

FIG. 3

EP 3 261 632 B1

FIG. 4

FIG. 5A

FIG. 5B

FIG. 5C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014079823 A
- US 5965596 A
- US 6426361 B
- US 7825084 B
- US 8067381 B
- US 8329207 B

**Non-patent literature cited in the description**

- **ARTIOLI G.G. ; GUALANO B. ; SMITH A. ; STOUT J.R. ; JUNIOR A.H.L.** The Role of β-alanine Supplementation on Muscle Carnosine and Exercise Performance. *Med Sci Sports Exerc,* 2010, vol. 42 (6), 1162-1173
- **BAGUET, A. et al.** Important role of muscle carnosine in rowing performance. *J of App. Physio.,* 2010, vol. 109 (4), 1096-1101
- **BOLDYREV A.A. ; STVOLINSKY S.L. ; FEDORO-VA T.N. ; SUSLINA Z.A.** Carnosine as a natural antioxidant and geroprotector: from molecular mechanisms to clinical trials. *Rejuvenation Res.,* 2010, vol. 13, 156-158
- **CASSLER N.M. ; SAMS R. ; CRIPE P.A. ; MCG-LYNN A.F. ; PERRY A.B. ; BANKS B.A.** Patterns and perceptions of supplement use by U.S. Marines deployed to Afghanistan. *Mil Med.,* 2013, vol. 178 (6), 659-64
- **DERAVE, W. et al.** Muscle carnosine metabolism and β-alaninesupplementation in relation to exercise and training. *Sports medicine,* 2010, vol. 40 (3), 247-263
- **DERAVE W. ; OZDEMIR M.S. ; HARRIS R.C. ; POT-TIER A. ; REYNGOUDT H. ; KOPPO K. ; WISE J.A. ; ACHTEN E.** Beta-Alanine supplementation augments muscle carnosine content and attenuates fatigue during repeated isokinetic contraction bouts in trained sprinters. *J Appl Physiol,* 2007, vol. 103 (5), 1736-1743
- **DUNNETT M. ; HARRIS R.C.** Influence of oral beta-alanine and L-histidine supplementation on the carnosine content of the gluteus medius. *Equine Vet J,* 1999, 499-504
- **DEL FAVORO S. ; ROSCHEL H. ; SOLIS M. ; HA-YASHI A. ; ARTIOLO G. ; OTADUY M. ; BENATTI F. ; HARRIS R. ; WISE J. ; LEITE C.** Beta-alanine (Carnosyn™) supplementation in elderly subjects (60-80 years): effects on muscle carnosine content and physical capacity. *Amino Acids,* 2012, vol. 43, 49-56
- **ESTRADA A. ; KELLEY A.M. ; WEBB C.M. ; ATHY J.R. ; CROWLEY J.S.** Modafinil as a replacement for dextroamphetamine for sustaining alertness in military helicopter pilots. *Aviat Space Environ Med.,* 2012, vol. 83 (6), 556-64
- **EVANS R.K. ; SCOVILLE C.R. ; ITO M.A. ; MELLO R.P.** Upper body fatiguing exercise and shooting performance. *Mil. Med.,* 2003, vol. 168, 451-456
- **GILLINGHAM R.L. ; KEEFE A.A. ; TIKUISIS P.** Acute caffeine intake before and after fatiguing exercises improves target shooting engagement time. *Aviat. Space Environ. Med.,* 2004, vol. 75, 865-871
- **GREEN S.B. ; SALKIND N.J. ; AKEY T.M.** Using SPSS for Windows: Analyzing and Understanding Data. Prentice Hall, 2000
- **HARMAN, E. A. et al.** Effects of two different eight-week training programs on military physical performance. *The Journal of Strength & Conditioning Research,* 2008, vol. 22 (2), 524-534
- **HARRIS R.C. ; TALLON M.J. ; DUNNETT M. ; BOOBIS L. ; COAKLEY J. ; KIM H.J. ; FALLOW-FIELD J.L. ; HILL C.A. ; SALE C. ; WISE J.A.** The absorption of orally supplied β-alanineand its effect on muscle carnosine synthesis in human vastus lateralis. *Amino Acids,* 2006, vol. 30 (3), 279-289
- **HAYMAN M.** Two minute clinical test for measurement of intellectual impairment in psychiatric disorders. *Arch Neuro Psychiatry,* 1942, vol. 47, 454-64
- **HILL C.A. ; HARRIS R.C. ; KIM H.J. ; HARRIS B.D. ; SALE C. ; BOOBIS L.H. ; KIM C.K. ; WISE J.A.** Influence of β-alaninesupplementation on skeletal muscle carnosine concentrations and high intensity cycling capacity. *Amino Acids,* 2007, vol. 32 (2), 225-233
- **HOBSON R.M. ; SAUNDERS B. ; BALL G. ; HAR-RIS R.C. ; SALE C.** Effects of Beta-alanine supplementation on exercise performance: a meta-analysis. *Amino Acids.,* 2012, vol. 43 (1), 25-37

- **HOFFMAN J.R. ; EMERSON N.S. ; STOUT J.R.** β-alaninesupplementation. *Curr. Sports Med. Rep.,* 2012, vol. 11, 189-195
- **HOFFMAN J.R. ; RATAMESS N.A. ; FAIGENBAUM A.D. ; ROSS R. ; KANG J. ; STOUT J.R. ; WISE J.A.** Short-duration β-alaninesupplementation increases training volume and reduces subjective feelings of fatigue in college football players. *Nutr Res,* 2008, vol. 28 (1), 31-35
- **HOFFMAN J.R. ; RATAMESS N.A. ; KANG J. ; MANGINE G. ; FAIGENBAUM A.D. ; STOUT J.R.** Effect of creatine and β-alanine supplementation on performance and endocrine responses in strength/power athletes. *Int J Sport Nutr Exerc Metabab,* 2006, vol. 16 (4), 430-446
- **HOFFMAN J.R. ; RATAMESS N.A. ; ROSS R. ; KANG J. ; MAGRELLI J. ; NEESE K. ; FAIGENBAUM A.D. ; WISE J.A.** β-Alanine and the hormonal response to exercise. *Int J Sports Med,* 2008, vol. 29 (12), 952-958
- **JORDAN T. ; LUKASZUK J. ; MISIC M. ; UMOREN J.** Effects of beta-alanine supplementation on the onset of blood lactate accumulation (OBLA) during treadmill running: Pre/post 2 treatment experimental design. *J Int Soci Sports Nutr,* 2010, vol. 7, 20-27
- **KENDRICK I.P. ; HARRIS R.C. ; KIM H.J. ; KIM C.K. ; DANG V.H. ; LAM T.Q. ; BUI T.T. ; SMITH M. ; WISE J.A.** The effects of 10 weeks of resistance training combined with beta-alanine supplementation on whole body strength, force production, muscular endurance and body composition. *Amino Acids.,* 2008, vol. 34 (4), 547-554
- **KERN B.D. ; ROBINSON T.L.** Effects of β-alaninesupplementation on performance and body composition in collegiate wrestlers and football players. *J Strength Cond Res.,* 2011, vol. 25, 1804-1815
- **KNAPIK, J. J. et al.** Soldier load carriage: historical, physiological, biomechanical, and medical aspects. *Military Medicine,* 2004, vol. 169 (1), 45-56
- **LIEBERMAN H.R. ; BATHALON G.P. ; FALCO C.M. ; KRAMER F.M. ; MORGAN C.A. 3RD ; NIRO P.** Severe decrements in cognition function and mood induced by sleep loss, heat, dehydration, and undernutrition during simulated combat. *Biol Psychiatry,* 2005, vol. 57, 422-429
- **LIEBERMAN H.R. ; STAVINOHA T.B. ; MCGRAW S.M. ; WHITE A. ; HADDEN L.S. ; MARRIOTT B.P.** Use of dietary supplements among active-duty US Army soldiers. *Am J Clin Nutr.,* 2010, vol. 92, 985-995
- **LIEBERMAN H.R. ; THARION W.J. ; SHUKITT-HALE B. ; SPECKMAN K.L. ; TULLEY R.** Effect of caffeine, sleep loss, and stress on cognitive performance and mood during U.S. Navy SEAL training. *Psychopharmacology,* 2002, vol. 164, 250-261
- **MURAKAMI T. ; FURUSE M.** The impact of taurine- and beta-alanine-supplemented diets on behavioral and neurochemical parameters in mice: antidepressant versus anxiolytic-like effects. *Amino Acids.,* 2010, vol. 39, 427-434
- **NIBBELING, N. et al.** The effects of anxiety and exercise-induced fatigue on shooting accuracy and cognitive performance in infantry soldiers. *Ergonomics(ahead-of-print),* 2014, 1-14
- **NINDL B.C. ; BARNES B.R. ; ALEMANY J.A. ; FRYKMAN P.N. ; SHIPPEE R.L. ; FRIEDL K.E.** Physiological consequences of U.S. army ranger training. *Med. Sci. Sports Exerc.,* 2007, vol. 39, 1380-1387
- **NINDL, B. C. et al.** Physiological Employment Standards III: physiological challenges and consequences encountered during international military deployments. *European journal of applied physiology,* 2013, vol. 113 (11), 2655-2672
- **NINDL, B. C. et al.** Physical performance responses during 72 h of military operational stress. *Medicine and science in sports and exercise,* 2002, vol. 34 (11), 1814-1822
- **RUSSO M.B. ; ARNETT M.V. ; THOMAS M.L. ; CALDWELL J.A.** Ethical use of cogniceuticals in the militaries of democratic nations. *Amer. J. Bioethics.,* 2008, vol. 8, 39-49
- **SOHER, B. J. et al.** Quantitative proton MR spectroscopic imaging of the human brain. *Magnetic resonance in medicine,* 1996, vol. 35 (3), 356-363
- **STELLINGWERFF, T. et al.** Effect of two β-alaninedosing protocols on muscle carnosine synthesis and washout. *Amino acids,* 2012, vol. 42 (6), 2461-2472
- **STOUT J.R. ; CRAMER J.T. ; MIELKE M. ; O'KROY J. ; TOROK D.J. ; ZOELLER R.F.** Effects of twenty-eight days of β-alanineand creatine monohydrate supplementation on the physical working capacity at neuromuscular fatigue threshold. *J Strength Cond Res,* 2006, vol. 20 (4), 928-931
- **STOUT J.R. ; CRAMER J.T. ; ZOELLER R.F. ; TOROK D. ; COSTA P. ; HOFFMAN J.R. ; HARRIS R.C. ; O'KROY J.** Effects of β-alanine supplementation on the onset of neuromuscular fatigue and ventilatory threshold in women. *Amino Acids,* 2007, vol. 32 (3), 381-386
- **STOUT J.R. ; GRAVES S.B. ; SMITH A.E. ; HARTMEN M.J. ; CRAMER J.T. ; BECK T.W. ; HARRIS R.C.** The effects of beta-alanine supplementation on neuromuscular fatigue in elderly (55-92 years): a double-blinded randomized study. *JISSN,* 2008, vol. 5 (21
- **VAN THIENEN R. ; VAN PROEYEN K. ; VANDEN EYNDE B. ; PUYPE J. ; LEFERE T. ; HESPEL P.** Beta-alanine, improves sprint performance in endurance cycling. *Med Sci Sports Exer,* 2009, vol. 41 (4), 898-903

- **VARLEY, M.C. ; FAIRWEATHER, I.H. ; AUGHEY, R.J.** Validity and reliability of GPS for measuring instantaneous velocity during acceleration, deceleration, and constant motion. *J Sports Sci,* 2012, vol. 30, 121-127
- **WEEKS S.R. ; MCAULIFFE C.L. ; DURUSSEL D. ; PASQUINA P.F.** Physiological and psychological fatigue in extreme conditions: The military example. *Phys. Med Rehab.,* 2010, vol. 2, 438-441
- **WELLS A.J. ; HOFFMAN J.R. ; GONZALEZ A.M. ; STOUT J.R. ; FRAGALA M.S. ; MANGINE G.T. ; MCCORMACK W.P. ; JAJTNER A.R. ; TOWNSEND J.R. ; ROBINSON IV, E.H.** A Supplement Containing Phosphatidylserine Attenuates Post-Exercise Mood Disturbance and Perception of Fatigue in Humans. *Nutrition Research.,* 2013, vol. 33, 464-472
- **WELSH T.T. ; ALEMANY J.A. ; MONTAIN S.J. ; FRYKMAN P.N. ; YOUNG A.J. ; NINDL B.C.** Effects of intensified military field training on jumping performance. *Int. J. Sports Med.,* 2008, vol. 29, 45-52
- Diagnostic and Statistical Manual of Mental Disorders. American Psychiatric Association, 1994
- Diagnostic and Statistical Manual of Mental Disorders. American Psychiatric Association, 2013
- **ANGELUCCI F ; RICCI V ; GELFO F ; MARTINOTTI G ; BRUNETTI M ; SEPEDE G ; SIGNORELLI M ; AGUGLIA E ; PETTORRUSO M ; VELLANTE F.** BDNF serum levels in subjects developing or not post-traumatic stress disorder after trauma exposure. *Brain and Cognition,* 2014, vol. 84, 118-122
- **BISSON JI ; SHEPHERD JP ; JOY D ; PROBERT R ; NEWCOMBE RG.** Early cognitive-behavioural therapy for post-traumatic stress symptoms after physical injury. Randomized controlled trial. *British Journal Psychiatry,* 2004, vol. 184, 63-69
- **BONFANTI L ; PERETTO P ; DE MARCHIS S ; FASOLO A.** Carnosine-related dipeptides in the mammalian brain. *Progress in Neurobiology,* 1999, vol. 59, 333-353
- **BRYANT RA ; CREAMER M ; O'DONNELL M ; SILOVE D ; MCFARLANE AC ; FORBES D.** A comparison of the capacity of DSM-IV and DSM-5 acute stress disorder definitions to predict posttraumatic stress disorder and related disorders. *Journal of Clinical Psychiatry,* 2014
- **BRYANT RA ; MASTRODOMENICO J ; FELMINGHAM KL ; HOPWOOD S ; KENNY L ; KANDRIS E ; CAHILL C ; CREAMER M.** Treatment of acute stress disorder: a randomized controlled trial. *Arch. Gen. Psychiatry.,* 2008, vol. 65, 659-703
- **CASTRÉN E ; RANTAMÄKI T.** The role of BDNF and its receptors in depression and antidepressant drug action: Reactivation of developmental plasticity. *Dev Neurobiol.,* 2010, vol. 70, 289-97

- **COBURN-LITVAK PS ; POTHAKOS K ; TATA DA ; MCCLOSKEY DP ; ANDERSON BJ.** Chronic administration of corticosterone impairs spatial reference memory before spatial working memory in rats. *Neurobiol Learn Mem.,* 2003, vol. 80, 11-23
- **COHEN H ; KOZLOVSKY N ; CRAMER A ; MATAR MA ; ZOHAR J.** Animal model for PTSD: From clinical concept to translational research. *Neuropharmacology,* 2012, vol. 62, 715-724
- **COHEN H ; LIU T ; KOZLOVSKY N ; KAPLAN Z ; ZOHAR J ; MATHÉ AA.** The neuropeptide Y (NPY)-ergic system is associated with behavioral resilience to stress exposure in an animal model of post-traumatic stress disorder. *Neuropsychopharmacology,* 2012, vol. 37, 350-363
- **COHEN H ; ZOHAR J.** An animal model of posttraumatic stress disorder: the use of cut-off behavioral criteria. *Annals of the New York Academy of Science,* 2004, vol. 1032, 167-178
- **COHEN H ; ZOHAR J ; GIDRON Y ; MATAR MA ; BELKIND D ; LOEWENTHAL U ; KOZLOVSKY N ; KAPLAN Z.** Blunted HPA axis response to stress influences susceptibility to posttraumatic stress response in rats. *Biol. Psychiatry.,* 2006, vol. 59, 1208-1218
- **COHEN H ; ZOHAR J ; MATAR M.** The relevance of differential response to trauma in an animal model of posttraumatic stress disorder. *Biol. Psychiatry.,* 2003, vol. 53, 463-473
- **COHEN H ; ZOHAR J ; MATAR M A ; ZEEV K ; LOEWENTHAL U ; RICHTER-LEVIN G.** Setting apart the affected: the use of behavioral criteria in animal models of post-traumatic stress disorder. *Neuropsychopharmacology,* 2004, vol. 29, 1962-1970
- **FUXE K ; CINTRA A ; AGNATI LF ; HARFSTRAND A ; WIKSTROM AC ; OKRET S ; ZOLI M ; MILLER LS ; GREENE JL ; GUSTAFSSON JA.** Studies on the cellular localization and distribution of glucocorticoid receptor and estrogen receptor immunoreactivity in the central nervous system of the rat and their relationship to the monoaminergic and peptidergic neurons of the brain. *Journal of Steroid Biochemistry,* 1987, vol. 27, 159-170
- **KAOUANE N ; PORTE Y ; VALLEE M ; BRAYDA-BRUNO L ; MONS N ; CALANDREAU L ; MARIGHETTO A ; PIAZZA PV ; DESMEDT A.** Glucocorticoids can induce PTSD-like memory impairements in mice. *Science,* 2012, vol. 335, 1510-1513
- **KIRKPATRICK HA ; HELLER GM.** Post-traumatic stress disorder: Theory and treatment update. *International Journal of Psychiatry in Medicine,* 2014, vol. 47, 337-346

- **KIM H ; YI JH ; CHOI K ; HONG S ; SHIN KS ; KANG SJ.** Regional differences in acute corticosterone-induced dendritic remodeling in the rat brain and their behavioral consequences. *BMC Neuroscience,* 2014, vol. 15, 65
- **KIM JJ ; FANSELOW MS ; DECOLA JP ; LANDEIRA-FERNANDEZ J.** Selective impairment of long-term but not short-term conditional fear by the N-methyl-D-aspartate antagonist APV. *Behavioral Neuroscience,* 1992, vol. 106, 591-596
- **KOHEN R ; YAMAMOTO Y ; CUNDY KC ; AMES BN.** Antioxidant activity of carnosine, homocarnosine, and anserine present in muscle and brain. *Proceedings of the National Academy of Sciences,* 1988, vol. 85, 3175-3179
- **KOZLOVSKY N ; MATAR MA ; KAPLAN Z ; KOTLER M ; ZOHAR J ; COHEN H.** Long-term down-regulation of BDNF mRNA in rat hippocampal CA1 subregion correlates with PTSD-like behavioral stress response. *International Journal of Neuropsychopharmacology,* 2007, vol. 10, 741-758
- **MATAR MA ; ZOHAR J ; COHEN H.** Translationally relevant modeling of PTSD in rodents. *Cell Tissue Research,* 2013, vol. 354, 127-139
- **MCEWEN BS.** Stress and hippocampal plasticity. *Annual Review of Neuroscience,* 1999, vol. 22, 105-122
- **MCEWEN BS.** Physiology and neurobiology of stress and adaptation: Central role of the brain. *Physiological Reviews,* 2007, vol. 87, 873-904
- **YEHUDA R ; WITTERT G.** Cortisol response to acute trauma and risk of posttraumatic stress disorder. *Psychoneuroendocrinology,* 2011, vol. 36, 720-727
- **MILAD M ; QUIRK GJ.** Neurons in medial prefrontal cortex signal memory for fear extinction. *Nature,* 2002, vol. 420, 70-74
- **MILLER MM ; MCEWEN BS.** Establishing an agenda for translational research on PTSD. *Annals of the New York Academy of Science,* 2006, vol. 1071, 294-312
- **MURAKAMI T ; FURUSE M.** The impact of taurine- and beta-alanine-supplemented diets on behavioral and neurochemical parameters in mice: antidepressant versus anxiolytic-like effects. *Amino Acids,* 2010, vol. 39, 427-434
- **MYERS B ; MCKLVEEN JM ; HERMAN JP.** Glucocorticoid actions on synapses, circuits, and behavior: Implications for the energetics of stress. *Frontiers in Neuroendocrinology,* 2014, vol. 35, 180-196
- **RICHARDSON MP ; STRANGE BA ; DOLAN RJ.** Encoding of emotional memories depends on amygdala and hippocampus and their interactions. *Nature Neuroscience,* 2004, vol. 7, 278-285

- **ROMEO RD ; WATERS EM ; MCEWEN BS.** Steroid-induced hippocampal synaptic plasticity; sex differences and similarities. *Neuron Glia Biology,* 2004, vol. 1, 219-29
- **SEBASTIAN V ; ESTIL JB ; CHEN D ; SCHROTT LM ; SERRANO PA.** Acute physiological stress promotes clustering of synaptic markers and alters spine morphology in the hippocampus. *Plos One,* 2013, vol. 8, e79077
- **SOLANKI N ; ALKADHI I ; ATROOZ F ; PATKI G ; SALIM S.** Grape powder prevents cognitive, behavioral, and biochemical impairments in a rat model of posttraumatic stress disorder. *Nutrition Research,* 2015, vol. 35, 65-75
- **SOUSA N ; MADEIRA MD ; PAULA-BARBOSA MM.** Effects of corticosterone treatment and rehabilitation on the hippocampal formation of neonatal and adult rats. An unbiased stereological study. *Brain Research,* 1998, vol. 794, 199-210
- **STEWART MG ; DAVIES HA ; SANDI C ; KRAEV IV ; ROGACHEVSKY VV ; PEDDIE CJ ; RODRIGUEZ JJ ; CORDERO MI ; DONOHUE HS ; GABBOTT PL.** Stress suppresses and learning induces plasticity in CA3 of rat hippocampus: a three dimensional ultrastructural study of thrny excrescences and their postsynaptic densities. *Neuroscience,* 2005, vol. 131, 43-54
- **TAKEUCHI K ; TOYOHAMA H ; SAKAQUCHI M.** A hyperosmotic stress-induced mRNA of carp cell encodes NA(+)- and Cl(-)-dependent high affinity taurine transporter. *Biochiica et Biophysica Acta,* 2000, vol. 1464, 219-230
- **TOMONAGA S ; KAJI Y ; TACHIBANA T ; DENBOW DM ; FURUSE M.** Oral administration of β-alanine modifies carnosine concentrations in the muscles and brains of chicken. *Animal Science Journal,* 2005, vol. 76, 249-254
- **TOMONAGA S ; MATSUMOTO M ; FURUSE M.** β-alanine enhances brain and muscle carnosine levels in broiler chicks. *The Journal of Poultry Science,* 2012, vol. 49, 308-312
- **TOMONAGA S ; TACHIBANA T ; TAKAGI T ; SAITO ES ; ZHANG R ; DENBOW DM ; FURUSE M.** Effect of central administration of carnosine and its constituents on behaviors in chicks. *Brain Research Bulletin,* 2004, vol. 63, 75-82
- **TOMONAGA S ; YAMANE H ; ONITSUKA E ; YAMADA S ; SATO M ; TAKAHATA Y ; MORIMATSU F ; FURUSE M.** Carnosine-induced anti-depressant-like activity in rats. *Pharmacology, Biochemistry and Behavior,* 2008, vol. 89, 627-632
- **WILSON CB ; MCLAUGHLIN LD ; NAIR A ; EBENEZER PJ ; DANGE R ; FRANCIS J.** Inflammation and oxidative stress are elevated in the brain, blood, and adrenal glands during the progression of post-traumatic stress disorder in a predator exposure animal model. *Plos One,* 2013, vol. 8, e76146

- **YAO SY ; LAU BW ; TONG JB ; WONG R ; CHING YP ; QUI G ; TANG SW ; LEE TMC ; SO KF.** Hippocampal neurogenesis and dendritic plasticity support running-improved spatial learning and depression-like behavior in stressed rats. *PloS One,* 2011, vol. 6, e24263

- **ZOHAR J ; YAHALOM H ; KOZLOVSKY N ; CWIKEL-HAMZANY S ; MATAR MA ; KAPLAN Z ; YEHUDA R ; COHEN H.** High dose hydrocortisone immediately after trauma may alter the trajectory of PTSD: Interplay between clinical and animal studies. *European Neuropsychopharmacology,* 2011, vol. 21, 796-809